# EUROPEAN PATENT SPECIFICATION

(11) **EP 1 490 058 B1**
(45) Date of publication and mention of the grant of the patent: **10.10.2007**
(21) Application number: 03706045.6
(22) Date of filing: 07.02.2003
(51) Int. Cl.: C07D 491/10, C07D 493/10, C07D 495/10, A01N 43/90, C07F 9/6561, C07D 309/10, C07D 309/14, C07D 309/08

(54) **PEROXISOME PROLIFERATOR ACTIVATED RECEPTOR MODULATORS**
MODULATOREN VON PEROXISOME PROLIFERATOR-AKTIVIERTEN REZEPTOREN
MODULATEURS DU RECEPTEUR ACTIVE PAR L'AGENT DE PROLIFERATION DU PEROXISOME

(30) Priority: 21.02.2002 US 359428 P
(43) Date of publication of application: 29.12.2004
(73) Proprietor: ELI LILLY AND COMPANY, Indianapolis, Indiana 46285 (US)
(72) Inventor: GIBSON, Tracey, Ann, Indianapolis, IN 46268 (US); MANTLO, Nathan, Bryan, Brownsburg, IN 46112 (US); THOMPSON, Richard, Craig, Frankfort, IN 46041 (US)
(74) Representative: Burnside, Ivan John
(86) International application number: PCT/US2003/003112
(87) International publication number: WO 2003/072099

(56) References cited:
- EP-A- 0 015 005
- EP-A- 0 157 225
- EP-A- 0 894 795
- EP-A- 1 167 357
- WO-A-00/64888
- WO-A-03/024937
- DE-A- 2 641 060
- US-A- 4 314 065

## Description

### BACKGROUND OF THE INVENTION

Peroxisome Proliferator Activated Receptors (PPARs) are members of the nuclear hormone receptor super family, which are ligand-activated transcription factors regulating gene expression. Various subtypes of PPARs have been discovered. These include PPARα, NUC1, PPARγ and PPARδ.

The PPARα receptor subtypes are reported to be activated by medium and long-chain fatty acids. They are involved in stimulating beta-oxidation of fatty acids and with the activity of fibrates which reportedly produce a substantial reduction in plasma triglycerides and moderate reduction in low-density lipoprotein (LDL) cholesterol.

PPARα, PPARγ and PPARδ receptors have been implicated in diabetes mellitus, cardiovascular disease, obesity, Syndrome X and gastrointestinal disease, such as, inflammatory bowel disease. Syndrome X is the combination of symptoms which include hyperinsulemia combined with hypertension, elevated body weight, elevated triglycerides and elevated LDL.

WO 03/024937 claiming a priority of 14 September 2001 and published 27 March 2003 is directed to Benzimidazolylalkoxyaryl alkanoic acid derivatives and their use as anti hyperglycemics; US 4,314,065 is directed to diarylamine derivatives as herbicides; EP 0157225 is directed to a process for the preparation of benzimidazole derivatives; EP 1167357 is directed to alpha-substituted carboxylic acid derivatives as therapeutic agents for diabetes mellitus; DE 2641060 is directed to β-lactam derivatives; EP 0015005 is directed to phenoxy derivatives and their use as herbicides; EP 0894795 is directed to benzimidazole compounds and their use as inhibitors of interleukin 1β; WO 00/64888 is directed to di-aryl acid derivatives as PPAR receptor ligands.

Current PPAR agonist treatment for Syndrome X relates to the use of thiazolidinediones (TZDs) or other insulin sensitivity enhancers (ISEs). TZDs are a class of PPAR gamma agonists which have been shown to increase the sensitivity of insulin sensitive cells. Increasing insulin sensitivity rather than the amount of insulin in the blood reduces the likelihood of hypoglycemic coma. However, TZDs and ISEs typically have little effect in preventing the cardiovascular part of Syndrome X in that their administration usually dose not result in the lowering of triglycerides and LDL-cholesterol while raising HDL cholesterol. Furthermore, side effects commonly associated with treatment with TZDs include significant weight gain, and, for troglitazone, liver toxicity. Therefore, a need exists for new pharmaceutical agents which affect treat or prevent cardiovascular disease, particularly that associated with Syndrome X, while preventing or minimizing weight gain, and more preferably while improving insulin sensitivity.

### SUMMARY OF THE INVENTION

The present invention is directed to compounds represented by the following structural 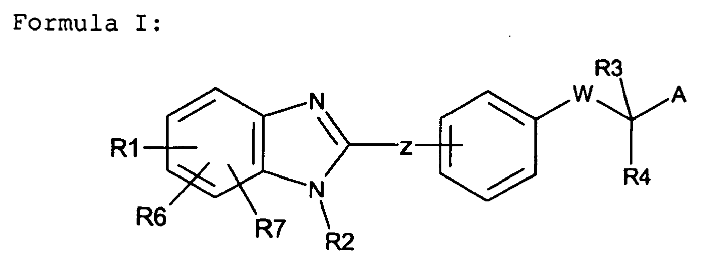 and pharmaceutically acceptable salts thereof, wherein:
(a) R1 is selected from the group consisting of hydrogen, C₁-C₈ alkyl, aryl-C₀₋₄-alkyl, heteroaryl-C₀₋₄-alkyl, and C3-C6 cycloalkylaryl-C₀₋₂-alkyl, wherein said C₁-C₈ alkyl, aryl-C₀₋₄-alkyl, heteroaryl-C₀₋₄-alkyl, and C3-C6 cycloalkylaryl-C₀₋₂-alkyl is each optionally substituted with from one to three substituents each independently selected from R1';
(b) R1', R2', R4', R6', A', Z' and R19' are each the group consisting of C₁-C₅ alkyl, C₁-C₅ alkoxy, C₁-C₅ haloalkyl, C₁-C₅ haloalkoxy, nitro, cyano, CHO, hydroxyl, C₁-C₄ alkanoic acid phenyl, aryloxy, SO₂R16, SR5, benzyloxy, alkylcarboxamido and COOH;
(c) R2 is selected from the group consisting of hydrogen, (C₂-C₄)alkyl-O-(C₂-C₄)alkyl-O-(C₁-C₄) alkyl, C₁-C₈ alkylene, aryl-C₀₋₄-alkyl, heteroaryl-C₀₋₄-alkyl, and C₃-C₆ cycloalkyl-C₀₋₄-alkyl, and wherein said (C₂-C₄) alkyl-O-(C₂-C₄)alkyl-O-(C₁-C₄) alkyl, C₁-C₈ alkylene, aryl-C₀₋₄-alkyl, heteroaryl-C₀₋₄-alkyl, and C₃-C₆ cycloalkyl-C₀₋₄-alkyl, is each optionally substituted with from one to three substituents each independently selected from R2';
(d) R3 is selected from the group consisting of hydrogen, C₁-C₅ alkyl, and C₁-C₅ alkoxy;
(e) R4 is selected from the group consisting of hydrogen, C₁-C₅ alkyl, C₁-C₅ alkoxy, C₃-C₆ cycloalkyl, and aryl C₀-C₄ alkyl, and wherein said C₁-C₅ alkyl, C₁-C₅ alkoxy, C₃-C₆ cycloalkyl, and aryl C₀-C₄ alkyl is each optionally substituted with from one to three substituents each independently selected from R4'; and wherein R3 and R4 are optionally combined to form a C₃-C₄ cycloalkyl;
(f) R5 and R16 are each selected from the group consisting of hydrogen, (C₁-C₆)alkyl and halo(C₁-C₆)alky;
(g) R6 and R7 are each independently selected from the group consisting of hydrogen, (C₁-C₆) alkyl, (C₁-C₆) alkenyl, halo (C₁-C₆) alkyl, halo, oxy, (C₁-C₆) alkoxy, and wherein said (C₁-C₆) alkyl, halo (C₁-C₆) alkyl, and (C₁-C₆) alkoxy are each is each optionally substituted with from one to three substituents each independently selected from R6'; and wherein R6 and R7 optionally combine to form a C3-C6 aryl that is fused to the group from which R6 and R7 each originate;
(h) W is selected from the group consisting of O, C, N and S;
(i) Z is C₃ alkyl optionally substituted with Z';
(j) A is selected from the group consisting of carboxyl, carboxamide, sulfonamide, acylsulfonamide, tetrazole, and (CH₂)ₙ COOR19, and wherein said sulfonamide, acylsulfonamide, and tetrazole is each optionally substituted with from one to three substituents each independently selected from A';
(k) n is 0, 1, 2 or 3; and
(l) R19 is selected from the group consisting of hydrogen, C1-C4alkyl and arylmethyl, wherein said alkyl and arylmethyl is each optionally substituted with from one to three substituents each independently selected from R19'.
The present invention is directed to compounds represented by the following structural 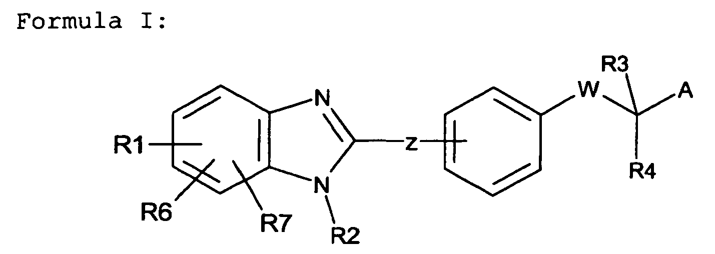 and pharmaceutically acceptable salts thereof, wherein:
(a) R1 is selected from the group consisting of hydrogen, substituted or unsubstituted group selected from C₁-C₈ alkyl, aryl-C₀₋₄-alkyl, heteroaryl-C₀₋₄-alkyl and C3-C6 cycloalkylaryl-C₀₋₂-alkyl;
(b) R2 is selected from the group consisting of hydrogen, substituted or unsubstituted group selected from (C₂-C₄) alkyl-O-(C₂-C₄)alkyl-O-(C₁-C₄) alkyl, C₁-C₈ alkylene, aryl-C₀₋₄-alkyl, heteroaryl-C₀₋₄-alkyl, and C₃-C₆ cycloalkyl-C₀₋₄-alkyl;
(c) R3 is selected from the group consisting of hydrogen, saturated or unsaturated C₁-C₅ alkyl, and C₁-C₅ alkoxy;
(d) R4 is selected from the group consisting of hydrogen, a substituted or unsubstituted group selected from C₁-C₅ alkyl, C₁-C₅ alkoxy, C₃-C₆ cycloalkyl, aryl C₀-C₄ alkyl and phenyl, or R3 and R4 are combined to form a C₃-C₄ cycloalkyl;
(e) R6 and R7 are each independently selected from the group consisting of hydrogen, substituted or unsubstituted (C₁-C₆) alkyl, halo(C₁-C₆) alkyl, halo, oxy, (C₁-C₆) alkoxy; wherein R6 and R7 optionally combine to form a C3-C6 aryl that is fused to the group from which R6 and R7 each originate;
(f) W is selected from the group consisting of O, C, N and S;
(g) Z is a straight C₃ alkyl optionally substituted by C₁-C₅ alkyl or C₁-C₅ alkoxy;
(h) A is an functional group selected from the group consisting of carboxyl, carboxamide, substituted or unsubstituted sulfonamide, substituted or unsubstituted acylsulfonamide and substituted or unsubstituted tetrazole, and (CH₂)ₙ COOR19;
(i) n is 0, 1, 2 or 3; and
(j) R19 is selected from the group consisting of hydrogen, optionally substituted C1-C4alkyl and optionally substituted arylmethyl.

In another feature of this invention, a compound claimed herein is radiolabeled.

It is generally more preferred that A is a carboxyl group. It is generally even more preferred that R₃ is H or CH₃. It may be preferred that both R3 and R4 are each CH₃. In another preferred embodiment, R3 and R4 are each hydrogen.

In one embodiment, the present invention also relates to pharmaceutical compositions which comprising at least one compound of the present invention, or a pharmaceutically acceptable salt thereof, and a pharmaceutically acceptable carrier.

In a further embodiment, the present invention relates to a method of making a compound represented by Structural Formula I.

The compounds of the present invention are believed to be effective in treating and preventing Syndrome X, Type II diabetes, hyperglycemia, hyperlipidemia, obesity, coagaulopathy, hypertension, atherosclerosis, and other disorders related to Syndrome X and cardiovascular diseases. In addition, the compounds can be associated with fewer clinical side effects than compounds currently used to treat these conditions. Further, compounds of this invention can be useful for lowering fibrinogen, increasing HDL levels, treating renal disease, controlling desirable weight, treating demyelinating diseases, treating certain viral infections, and treating liver disease.

### DETAILED DESCRIPTION OF THE INVENTION

The terms used to describe the instant invention have the following meanings herein.

As used herein, alkyl groups include straight chained or branched hydrocarbons, which are completely saturated.

As used herein, alkenyl groups are hydrocarbon chains having the indicated number of carbon atoms (branched or straight) and having at least one point of unsaturation, forming a double bond at such point of unsaturation.

Cycloalkyl groups, as used herein, include cyclic hydrocarbons, which are partially or completely saturated. As used herein, aryl groups include carbocyclic aromatic ring systems (e.g. phenyl), fused polycyclic aromatic ring systems (e.g. naphthyl and anthracenyl).and aromatic ring systems fused to carbocyclic non-aromatic ring systems (e.g., 1,2,3,4-tetrahydronaphthyl and benzodioxyl).

Heterocyclic group, as used herein, is a ring system having at least one heteroatom such as nitrogen, sulfur or oxygen. Heterocyclic groups include benzofuranyl, benzothiazolyl, benzothienyl, isoquinolyl, isoxazolyl, morpholino, oxadiazolyl, pyridyl, pyrimidinyl, pyrrolyl, quinolyl, tetrahydropyranyl and thienyl.

Suitable substituents when at least one of said R1, R2, R3, R4, R6, R7, A and R19 is substituted is one or more independently selected from the group consisting C₁-C₅ alkyl, C₁-C₅ alkoxy, C₁-C₅ haloalkyl, C₁-C₅ haloalkoxy, nitro, cyano, CHO, hydroxyl, C₁-C₄ alkanoic acid phenyl, aryloxy, SO₂R7, SR5, benzyloxy, alkylcarboxamido and COOH. R5 is an alkyl or a haloalkyl. When R1, R2, R3, R4, R6, R7, A or R19 is substituted, it is preferred that there are from 1-3 substitutions on said R1, R2, R3, R4, R6, R7, A and R19 group.

In one preferred embodiment of this invention, Z is unsubstituted.

Preferably, for the compounds of the present invention, represented by Structural Formula I, and with their respective pharmaceutical compositions, W is an oxygen.

When a compound represented by Structural Formula I has more than one chiral substituent it may exist in diastereoisomeric forms. The diastereoisomeric pairs may be separated by methods known to those skilled in the art, for example chromatography or crystallization and the individual enantiomers within each pair may be separated using methods familiar to the skilled artisan. The present invention includes each diastereoisomer of compounds of Structural Formula I and mixtures thereof.

Certain compounds of Structural Formula I may exist in different stable conformational forms which may be separable. Torsional asymmetry due to restricted rotation about an asymmetric single bond, for example because of steric hindrance or ring strain, may permit separation of different conformers. The present invention includes each conformational isomer of compounds of Structural Formula I and mixtures thereof.

Certain compounds of Structural Formula I may exist in zwitterionic form and the present invention includes each zwitterionic form of compounds of Structural Formula I and mixtures thereof.

"Pharmaceutically-acceptable salt" refers to salts of the compounds of the Structural Formula I which are substantially non-toxic to mammals. Typical pharmaceutically-acceptable salts include those salts prepared by reaction of the compounds of the present invention with a mineral or organic acid or an organic or inorganic base. Such salts are known as base addition salts, respectively. It should be recognized that the particular counterion forming a part of any salt of this invention is not of a critical nature, so long as the salt as a whole is pharmaceutically acceptable and as long as the counterion does not contribute undesired qualities to the salt as a whole.

By virtue of its acidic moiety, a compound of Structural Formula I forms salts with pharmaceutically acceptable bases.

Compounds of Structural Formula I, which are substituted with a basic group, may exist as salts with pharmaceutically acceptable acids. The present invention includes such salts. These salts may be prepared by methods known to those skilled in the art.

The term, "active ingredient" means the compounds generically described by Structural Formula I as well as the salts of such compounds.

The term "pharmaceutically acceptable" means that the carrier, diluent, excipients and salt must be compatible with the other ingredients of the composition, and not deleterious to the recipient thereof. Pharmaceutical compositions of the present invention are prepared by procedures known in the art using well known and readily available ingredients.

"Preventing" refers to reducing the likelihood that the recipient will incur or develop any of the pathological conditions described herein. The term "preventing" is particularly applicable to a patient that is susceptible to the particular patholical condition.

"Treating" refers to mediating a disease or condition and preventing, or mitigating, its further progression or ameliorate the symptoms associated with the disease or condition.

"Pharmaceutically-effective amount" means that amount of a compound, or of its salt thereof, that will elicit the biological or medical response of a tissue, system, or mammal. Such an amount can be administered prophylactically to a patient thought to be susceptible to development of a disease or condition. Such amount when administered prophylactically to a patient can also be effective to prevent or lessen the severity of the mediated condition. Such an amount is intended to include an amount which is sufficient to modulate a selected PPAR receptor or to prevent or mediate a disease or condition. Conditions prevented or treated by modulation of one or more PPAR receptors include diabetes mellitus, cardiovascular disease, Syndrome X, obesity and gastrointestinal disease.

A "mammal" is an individual animal that is a member of the taxonomic class Mammalia. The class Mammalia includes humans, monkeys, chimpanzees, gorillas, cattle, swine, horses, sheep, dogs, cats, mice, and rats.

Administration to a human is most preferred. The compounds and compositions of the present invention are useful for the treatment and/or prophylaxis of cardiovascular disease, for raising serum HDL cholesterol levels, for lowering serum triglyceride levels and for lower serum LDL cholesterol levels. Elevated triglyceride and LDL levels, and low HDL levels, are risk factors for the development of heart disease, stroke, and circulatory system disorders and diseases.

The compounds and compositions of the present invention are also useful for treating and/or preventing obesity.

Further, these compounds and compositions are useful for the treatment and/or prophylaxis of non-insulin dependent diabetes mellitus (NIDDM) with reduced or no body weight gains by the patients. Furthermore, the compounds and compositions of the present invention are useful to treat or prevent acute or transient disorders in insulin sensitivity, such as sometimes occur following surgery, trauma, myocardial infarction, and the like. The physician of ordinary skill will know how to identify humans who will benefit from administration of the compounds and compositions of the present invention.

The present invention further provides a method for the treatment and/or prophylaxis of hyperglycemia in a human or non-human mammal which comprises administering an effective, non-toxic amount of a compound of the general formula (I), or a tautomeric form thereof and/or a pharmaceutically acceptable salt thereof to a hyperglycemic human or non-human mammal in need thereof.

The invention also relates to the use of a compound of Formula I as described above, for the manufacture of a medicament for treating a PPAR receptor mediated condition.

A therapeutically effective amount of a compound of Structural Formula I can be used for the preparation of a medicament useful for treating Syndrome X, diabetes, treating obesity, lowering tryglyceride levels, lowering serum LDL levels, raising the plasma level of high density lipoprotein, and for treating, preventing or reducing the risk of developing atherosclerosis, and for preventing or reducing the risk of having a first or subsequent atherosclerotic disease event in mammals, particularly in humans. In general, a therapeutically effective amount of a compound of the present invention typically reduces serum triglyceride levels of a patient by about 20% or more, and increases serum HDL levels in a patient. Preferably, HDL levels will be increased by about 30% or more. In addition, a therapeutically effective amount of a compound, used to prevent or treat NIDDM, typically reduces serum glucose levels, or more specifically HbA1c, of a patient by about 0.7% or more.

Advantageously, compositions containing the compound of Structural Formula I or the salts thereof may be provided in dosage unit form, preferably each dosage unit containing from about 1 to about 500 mg be administered although it will, of course, readily be understood that the amount of the compound or compounds of Structural Formula I actually to be administered will be determined by a physician, in the light of all the relevant circumstances.

When used herein Syndrome X includes pre-diabetic insulin resistance syndrome and the resulting complications thereof, insulin resistance, non-insulin dependent diabetes, dyslipidemia, hyperglycemia obesity, coagulopathy, hypertension and other complications associated with diabetes. The methods and treatments mentioned herein include the above and encompass the treatment and/or prophylaxis of any one of or any combination of the following: pre-diabetic insulin resistance syndrome, the resulting complications thereof, insulin resistance, Type II or non-insulin dependent diabetes, dyslipidemia, hyperglycemia, obesity and the complications associated with diabetes including cardiovascular disease, especially atherosclerosis.

The compositions are formulated and administered in the same general manner as detailed herein. The compounds of the instant invention may be used effectively alone or in combination with one or more additional active agents depending on the desired target therapy. Combination therapy includes administration of a single pharmaceutical dosage composition which contains a compound of Structural Formula I and one or more additional active agents, as well as administration of a compound of Structural Formula I and each active agent in its own separate pharmaceutical dosage formulation. For example, a compound of Structural Formula I or thereof and an insulin secretogogue such as biguanides, thiazolidinediones, sulfonylureas, insulin, or α-glucosidose inhibitors can be administered to the patient together in a single oral dosage composition such as a tablet or capsule, or each agent administered in separate oral dosage formulations. Where separate dosage formulations are used, a compound of Structural Formula I and one or more additional active agents can be administered at essentially the same time, i.e., concurrently, or at separately staggered times, i.e., sequentially; combination therapy is understood to include all these regimens.

An example of combination treatment or prevention of atherosclerosis may be wherein a compound of Structural Formula I or salts thereof is administered in combination with one or more of the following active agents: antihyperlipidemic agents; plasma HDL-raising agents; antihypercholesterolemic agents, fibrates, vitamins, aspirin, and the like. As noted above, the compounds of Structural Formula I can be administered in combination with more than one additional active agent.

Another example of combination therapy can be seen in treating diabetes and related disorders wherein the compounds of Structural Formula I, salts thereof can be effectively used in combination with, for example, sulfonylureas, biguanides, thiazolidinediones, α-glucosidase inhibitors, other insulin secretogogues, insulin as well as the active agents discussed above for treating atherosclerosis.

The compounds of the present invention, and the pharmaceutically acceptable salts, have valuable pharmacological properties and can be used in pharmaceutical compositions containing a therapeutically effective amount of a compound of the present invention, or pharmaceutically acceptable salts thereof, in combination with one or more pharmaceutically acceptable excipients. Excipients are inert substances such as, without limitation carriers, diluents, fillers, flavoring agents, sweeteners, lubricants, solubilizers, suspending agents, wetting agents, binders, disintegrating agents, encapsulating material and other conventional adjuvants. Proper formulation is dependent upon the route of administration chosen. Pharmaceutical compositions typically contain from about 1 to about 99 weight percent of the active ingredient which is a compound of the present invention.

Preferably, the pharmaceutical formulation is in unit dosage form. A "unit dosage form" is a physically discrete unit containing a unit dose, suitable for administration in human subjects or other mammals. For example, a unit dosage form can be a capsule or tablet, or a number of capsules or tablets. A "unit dose" is a predetermined quantity of the active compound of the present invention, calculated to produce the desired therapeutic effect, in association with one or more pharmaceutically-acceptable excipients. The quantity of active ingredient in a unit dose may be varied or adjusted from about 0.1 to about 1000 milligrams or more according to the particular treatment involved.

The dosage regimen utilizing the compounds of the present invention is selected by one of ordinary skill in the medical or veterinary arts, in view of a variety of factors, including, without limitation, the species, age, weight, sex, and medical condition of the recipient, the severity of the condition to be treated, the route of administration, the level of metabolic and excretory function of the recipient, the dosage form employed, the particular compound and salt thereof employed, and the like.

Preferably, the compounds of the present invention are administered in a single daily dose, or the total daily dose may be administered in divided doses, two, three, or more times per day. Where delivery is via transdermal forms, of course, administration is continuous.

Suitable routes of administration of pharmaceutical compositions of the present invention include, for example, oral, eyedrop, rectal, transmucosal, topical, or intestinal administration; parenteral delivery (bolus or infusion), including intramuscular, subcutaneous, intramedullary injections, as well as intrathecal, direct intraventricular, intravenous, intraperitoneal, intranasal, or intraocular injections. The compounds of the invention can also be administered in a targeted drug delivery system, such as, for example, in a liposome coated with endothelial cell-specific antibody.

For oral administration, the compounds can be formulated readily by combining the active compounds with pharmaceutically acceptable carriers well known in the art. Such carriers enable the compounds of the invention to be formulated as tablets, pills, powders, sachets, granules, dragees, capsules, liquids, elixirs, tinctures, gels, emulsions, syrups, slurries, suspensions and the like, for oral ingestion by a patient to be treated. Pharmaceutical preparations for oral use can be obtained by combining the active compound with a solid excipient, optionally grinding a resulting mixture, and processing the mixture of granules, after adding suitable auxiliaries, if desired, to obtain tablets or dragee cores.

For oral administration in the form of a tablet or capsule, the active ingredient may be combined with an oral, non-toxic, pharmaceutically-acceptable carrier, such as, without limitation, lactose, starch, sucrose, glucose, methyl cellulose, calcium carbonate, calcium phosphate, calcium sulfate, sodium carbonate, mannitol, sorbitol, and the like; together with, optionally, disintegrating agents, such as, without limitation, cross-linked polyvinyl pyrrolidone, maize, starch, methyl cellulose, agar, bentonite, xanthan gum, alginic acid, or a salt thereof such as sodium alginate, and the like; and, optionally, binding agents, for example, without limitation, gelatin, acacia, natural sugars, beta-lactose, corn sweeteners, natural and synthetic gums, acacia, tragacanth, sodium alginate, carboxymethyl-cellulose, polyethylene glycol, waxes, and the like; and, optionally, lubricating agents, for example, without limitation, magnesium stearate, sodium stearate, stearic acid, sodium oleate, sodium benzoate, sodium acetate, sodium chloride, talc, and the like. When a dosage unit form is a capsule, it may contain, in addition to materials of the above type, a liquid carrier such as a fatty oil.

Solid form formulations include powders, tablets and capsules. A solid carrier can be one or more substance which may also act as flavoring agents, lubricants, solubilisers, suspending agents, binders, tablet disintegrating agents and encapsulating material.

In powders, the carrier is a finely divided solid which is in admixture with the finely divided active ingredient. In tablets, the active ingredient is mixed with a carrier having the necessary binding properties in suitable proportions and compacted in the shape and size desired.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Sterile liquid formulations include suspensions, emulsions, syrups, and elixirs. The active ingredient can be dissolved or suspended in a pharmaceutically acceptable carrier, such as sterile water, sterile organic solvent, or a mixture of both sterile water and sterile organic solvent.

The active ingredient can also be dissolved in a suitable organic solvent, for example, aqueous propylene glycol. Other compositions can be made by dispersing the finely divided active ingredient in aqueous starch or sodium carboxymethyl cellulose solution or in a suitable oil.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

All formulations for oral administration should be in dosages suitable for such administration. Particularly suitable compositions for oral administration are unit dosage forms such as tablets and capsules.

For parental administration the compounds of the present invention, or salts thereof, can be combined with sterile aqueous or organic media to form injectable solutions or suspensions. Formulations for injection may be presented in unit dosage form, such as in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that each syringability exists. It must be stable under the conditions of manufacture and storage and must be preserved against any contamination. The carrier can be solvent or dispersion medium containing, for example, water, preferably in physiologically compatible buffers such as Hanks's solution, Ringer's solution, or physiological saline buffer, ethanol, polyol (e.g. glycerol, propylene glycol and liquid polyethylene glycol), propylene glycol and liquid polyethylene glycol), suitable mixtures thereof, and vegetable oils. Under ordinary conditions of storage and use, these preparations contain a preservative to prevent the growth of microorganisms.

For transmucosal administration, penetrants appropriate to the barrier to be permeated are used in the formulation. Such penetrants are generally known in the art. The active compounds can also be administered intranasally as, for example, liquid drops or spray.

For buccal administration, the compositions may take the form of tablets or lozenges formulated in a conventional manner.

Pharmaceutical compositions of the present invention can be manufactured in a manner that is itself known, e.g., by means of conventional mixing, dissolving, granulating, dragee-making, levigating, emulsifying, encapsulating, entrapping or lyophilizing processes.

The following pharmaceutical formulations 1 and 2 are illustrative only and are not intended to limit the scope of the invention in any way. "Active Ingredient", refers to a compound according to Structural Formula I or salts thereof.

### Formulation 1

Hard gelatin capsules are prepared using the following ingredients:

| | Quantity |
|---|---|
| | (mg/capsule) |
| Active Ingredient | 250 |
| Starch, dried | 200 |
| Magnesium stearate | 10 |
| Total | 460 mg |

### Formulation 2

A tablet is prepared using the ingredients below:

| | Quantity |
|---|---|
| | (mg/tablet) |
| Active Ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg.

In yet another embodiment of the compounds of the present invention, the compound is radiolabelled, such as with carbon-14, or tritiated. Said radiolabelled or tritiated compounds are useful as reference standards for in vitro assays to identify new selective PPAR receptor agonists.

The compounds of the present invention can be useful for modulating insulin secretion, treating and/or preventing cardiovascular disease and as research tools. Certain compounds and conditions within the scope of this invention are preferred. The following conditions, invention embodiments, and compound characteristics listed in tabular form are preferred features and may be independently combined to produce a variety of preferred compounds and process conditions. The following list of embodiments of this invention is not intended to limit the scope of this invention in any way.

Some prefered characteristics of compounds of formula I are:
(a) R1 is selected from the group consisting of arylC₀-C₄alkyl and alkyl;
(b) R3 is methyl;
(c) R4 is hydrogen;
(d) R2 is hydrogen;
(e) R2 is C₁-C₆ alkylene;
(f) R2 is selected from the group consisting of (C₂-C₄)alkyl-O-(C₂-C₄)alkyl-O-(C₁-C₄) alkyl, aryl-C₀₋₄-alkyl, and C₃-C₆ cycloalkyl-C₀₋₄-alkyl;
(g) R2 is aryl-C₁₋₄-alkyl;
(h) Aryl is phenyl;
(i) R6 and R7 are each hydrogen;
(j) R6 and R7 combine to form a C3-C6 aryl that is fused to the group from which R6 and R7 each originate;
(k) W is O;
(l) W is C;
(n) z is substituted with one group selected from Z' ;
(o) A is selected from the group consisting of carboxyl, acylsulfonamide, tetrazole, and (CH₂)ₙ COOR19;
(p) A is (CH₂)ₙ COOR19;
(q) A is carboxyl;
(r) A compound of this invention is used to treat or prevent a condition that is at least in part associated with modulation of a PPAR receptor;
(s) A compound of this invention is used to treat or prevent atherosclerosis, dislipidemia, and/or another cardiovascular disease in a patient in need thereof; and
(t) A compound of this invention is formulated for oral administration.

### SYNTHESIS

Compounds of the present invention have been formed as specifically described in the examples. Further, many compounds are prepared as more generally as shown in the following schematic. Alternative synthesis methods may also be effective and known to the skilled artisan.

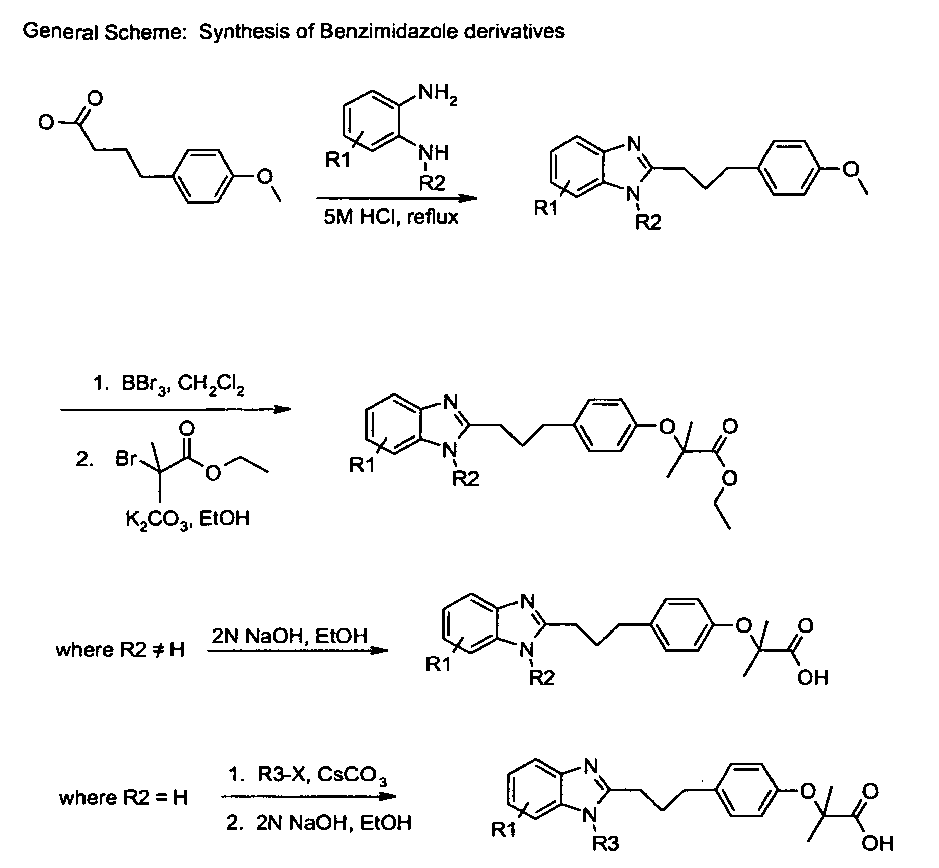

### EXEMPLIFICATION

The Examples provided herein are illustrative of the invention claimed herein

### Instrumental Analysis

Infrared spectra are recorded on a Perkin Elmer 781 spectrometer. ¹H NMR spectra are recorded on a Varian 400 MHz spectrometer at ambient temperature. Data are reported as follows: chemical shift in ppm from internal standard tetramethylsilane on the δ scale, multiplicity (b = broad, s = singlet, d = doublet, t = triplet, q = quartet, qn = quintet and m = multiplet), integration, coupling constant (Hz) and assignment. ¹³C NMR are recorded on a Varian 400 MHz spectrometer at ambient temperature. Chemical shifts are reported in ppm from tetramethylsilane on the δ scale, with the solvent resonance employed as the internal standard (CDCl₃ at 77.0 ppm and DMSO-d₆ at 39.5 ppm). High resolution mass spectra are obtained on VG ZAB 3F or VG 70 SE spectrometers. All analytical methods are performed using methods known to the skilled artisan, unless noted.

### Exemplified Compounds

### Example 1:

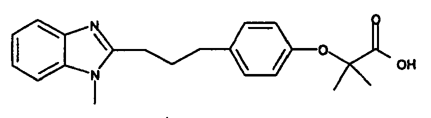

### Step A:

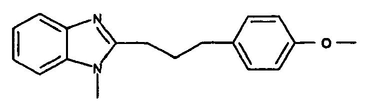

The 4-(4-methoxyphenyl) butyric acid (5 g, 0.026 mol) is combined with *N*-methyl-1,2-phenylene diamine (2.68 ml, 0.119 mol) in 50 ml of 5M HCl. The reaction is refluxed overnight. The pH of the solution is adjusted to pH=7 using 5% NaOH. The solution is then extracted with ethyl acetate. The organic layer is washed with 1M K₂CO₃ and brine. The solvent is concentrated to afford the desired product as a dark oil (5.43 g, 76%).
C₁₈H₂₀N₂O (MW = 280.16); mass spectroscopy (FD) = 280.3

### Step B:

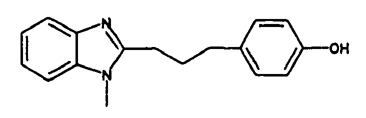

Boron tribromide (5.39 ml, 0.057 mol) is added to methylene chloride and cooled to 0°C. The methyl ether from **Step A** (5.43 g, 0.019 mol) is added dropwise over a fifteen-minute period. The reaction is warmed to room temperature. A solution of 1:1 methylene chloride and methanol is added to quench the reaction. After stirring for some time, the solvent is concentrated. Upon the addition of ethyl acetate, the product precipitated from the solution as a purple solid (3.26 g, 63%). The solid is collected by filtration and carried forth without further purification. C₁₇H₁₈N₂O (MW = 266.14)

### Step C:

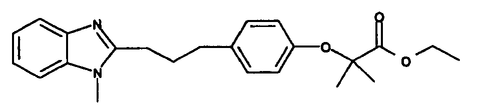

The phenol from **Step B** (3.24 g, 0.0122 mol) is dissolved in absolute ethanol (20 ml) and treated with K₂CO₃ (5.00 g, 0.0360 mol) followed by ethyl 2-bromoisobutyrate (8.95 ml, 0.0609 mol). The reaction is stirred at 80°C. Additional isobutyrate (8.95 ml) and K₂CO₃ (2.5g) is added to the reaction. Upon cooling, the reaction mixture is filtered then concentrated. The resulting residue is redissolved in methylene chloride and washed with water then brine. Purification by flash chromatography (1:1 hexanes: ethyl acetate) yields the ester (2.8 g, 60%).
C₂₃H₂₈N₂O₃ (MW = 380.21); mass spectroscopy (MH⁺) = 381.1

### Step D:

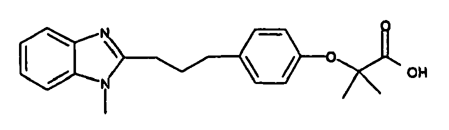

The ester from **Step C** (1 g, 0.0026 mol) is dissolved in ethanol (21 ml) and 2N NaOH (10 ml) is added. The reaction is refluxed for one hour. Water (50 ml) is added to the mixture and the pH is adjusted to pH = 6 using 5M HCl and ammonia in methanol. The desired product precipitated out of solution as a white solid (0.500 g, 54%) and is filtered then dried.
C₂₁H₂₄N₂O₃ (MW = 352.18); mass spectroscopy (MH⁺) = 353.3

### Example 2:

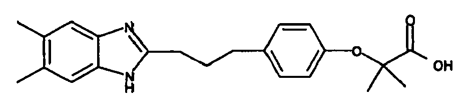

### Step A:

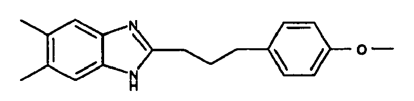

The 4-(4-methoxyphenyl) butyric acid (2 g, 0.010 mol) is combined with 4, 5-dimethyl-1, 2-phenylene diamine (1.36 ml, 0.010 mol) in 20 ml of 5M HCl. The reaction is refluxed overnight. The pH of the solution is adjusted to pH=7. The solution is then extracted with ethyl acetate. The organic layer is washed with 1M K₂CO₃ and brine. The solvent is concentrated to afford the desired product as a maroon solid (1.14 g, 39 %).
C₁₉H₂₂N₂O (MW = 294.17) ; mass spectroscopy (MH⁺) = 295.3

### Step B:

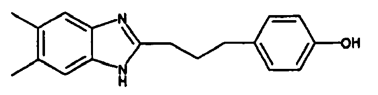

Boron tribromide (1.10 ml, 0.0116 mol) is added to methylene chloride and cooled to 0°C. The methyl ether from **Step A** (1.14 g, 0.0039 mol) is added; drop wise, over a fifteen minute period. The reaction is stirred for one hour. A solution of 1:1 methylene chloride and methanol (14 mL) is added to quench the reaction. After stirring for some time, the solvent is concentrated. The crude residue is redissolved in ethyl acetate and washed with water then brine. The organic layer is concentrated to afford the desired phenol (0.290 g, 27 %).
C₁₈H₂₀N₂O (MW = 280.16); mass spectroscopy (MH⁺) = 281.2

### Step C:

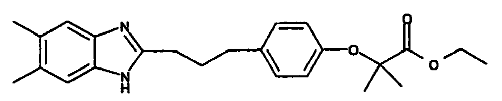

The phenol from **Step B** (0.164 g, 0.0059 mol) is dissolved in absolute ethanol (10 ml) and treated with K₂CO₃ (0.244 g, 0.00177 mol) followed by ethyl 2-bromoisobutyrate (0.430 ml, 0.0029 mol). The reaction is stirred at 76°C overnight. Additional isobutyrate (0.43 ml) is added to drive the reaction. Upon cooling, the reaction mixture is filtered then concentrated. Purification by flash chromatography (1:1 hexanes: ethyl acetate) yields the ester as a yellow oil (0.118 g, 51%).
C₂₄H₃₀N₂O₃ (MW = 394.23) ; mass spectroscopy (MH⁺) = 395.4

### Step D:

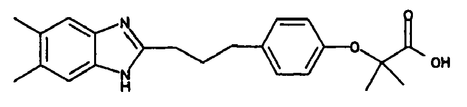

The ester from **Step C** (0.117 g, 0.000297 mol) is dissolved in ethanol (2 ml) and 2N NaOH (1 ml) is added. The reaction is refluxed for thirty minutes. Water (5 ml) is added to the mixture and the pH is adjusted to pH = 7 using 1N HCl. The desired product precipitated out of solution as a white solid (0.080 g, 73 %) and is filtered then dried. C₂₂H₂₆N₂O₃ (MW = 366.19); mass spectroscopy (MH⁺) = 367.1

### Example 3:

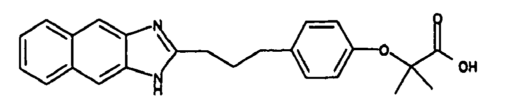

### Step A:

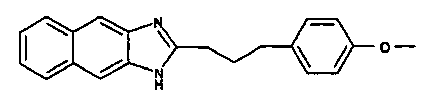

The 4-(4-methoxyphenyl) butyric acid (3.60 g, 0.0186 mol) is combined with 2,3-diamino-napthalene (3 g, 0.0189 mol) in 40 ml of 5M HCl. The reaction is refluxed overnight. The pH of the solution is adjusted to pH=7 using 5% NaHCO₃.
The solution is then extracted with ethyl acetate. The organic layer is washed with
1M K₂CO₃ and brine. The solvent is concentrated to afford the desired product as a brown solid (3.42 g, 58 %).
C₂₁H₂₀N₂O (MW = 316.16); mass spectroscopy (MH⁺) = 317.3

### Step B:

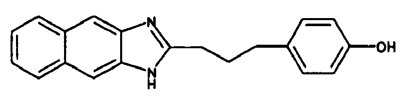

Boron tribromide (3.00 ml, 0.032 mol) is added to methylene chloride (60 ml) and cooled to 0°C. The methyl ether from **Step A** (3.42 g, 0.011 mol) is added slowly. The reaction is warmed to room temperature. A solution of 1:1 methylene chloride and methanol (42 ml) is added to quench the reaction. After stirring for some time, the solvent is concentrated. The resulting material is redissolved in ethyl acetate and washed with water. The organic layer is concentrated to afford the product (2.59 g, 78%).
C₂₀H₁₈N₂O (MW = 302.14); mass spectroscopy (MH⁺) = 303.0

### Step C:

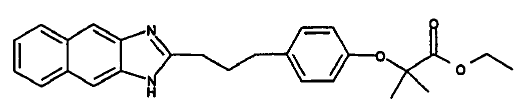

The phenol from **Step B** (1.50 g, 0.0050 mol) is dissolved in absolute ethanol (10 ml) and treated with K₂CO₃ (2.07 g, 0.0150 mol) followed by ethyl 2-bromoisobutyrate (7.33 ml, 0.050 mol). The reaction is stirred at 75 °C. Upon cooling, the reaction mixture is filtered then concentrated. Purification by flash chromatography (1:1 hexanes: ethyl acetate) yields the ester (1.09 g, 52 %).
C₂₆H₂₈N₂O₃ (MW = 416.21); mass spectroscopy (MH⁺) = 417.1

### Step D:

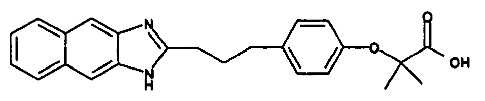

The ester from **Step C** (0.700 g, 0.00168 mol) is dissolved in ethanol (14 ml) and 2N NaOH (7 ml) is added. The reaction is refluxed for one hour. Water is added to the mixture and the pH is adjusted to pH = 6. The desired product precipitated out of solution as a tan solid (0.52 g, 80%) and is filtered then dried.
C₂₄H₂₄N₂O₃ (MW = 388.18) ; mass spectroscopy (MH⁺) = 389.2

### Example 4:

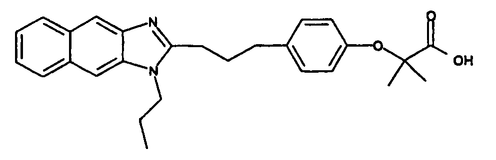

### Step A:

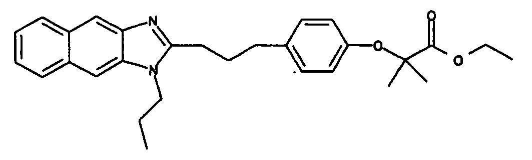

The ester from **Example 3, Step C** (0.144 g, 0.00035 mol) is dissolved in DMF (5 ml) and treated with CsCO₃ (0.282 g, 0.00087 mol) followed by 1-iodo-propane(0.057 g, 0.00180 mol). The reaction is stirred for forty-five minutesat 67°C. Ether is added to the reaction which is then extracted with water and brine. Purification by flash chromatography (3:1 hexanes: ethyl acetate) yields the ester (0.025 g, 16 %).
C₂₉H₃₄N₂O₃ (MW = 458.26); mass spectroscopy (MH⁺) = 459.4

### Step B:

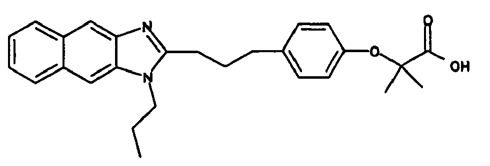

The ester from **Step A** (0.025 g, 0.0000545 mol) is dissolved in ethanol (4 ml) and 2N NaOH (2 ml) is added. The reaction is refluxed for thirty minutes. Water is added to the mixture and the pH is adjusted to pH = 6 using 1N HCl. The aqueous layer is extracted with ethyl acetate. The organic layer is concentrated to yield the desired acid (0.0148 g, 65 %).
C₂₇H₃₀N₂O₃ (MW = 430.23); mass spectroscopy (MH⁺) = 431.2

### Example 5:

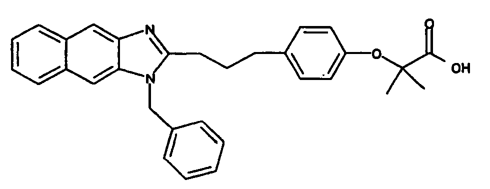

### Step A:

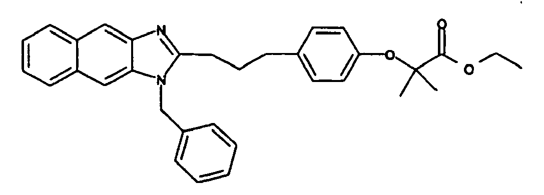

The ester from **Example 3, Step C** (0.144 g, 0.00035 mol) is dissolved in DMF (5 ml) and treated with CsCO₃ (0.282 g, 0.00087 mol) followed by benzyl bromide (0.066 ml, 0.00055 mol). The reaction is stirred for one hour at 67°C. Ether is added to the reaction and the layer is washed with water then brine. Purification by flash chromatography (4:1 hexanes: ethyl acetate) yields the ester (0.078 g, 44 %). C₃₃H₃₄N₂O₃ (MW = 506.26) ; mass spectroscopy (MH⁺) = 507.0

### Step B:

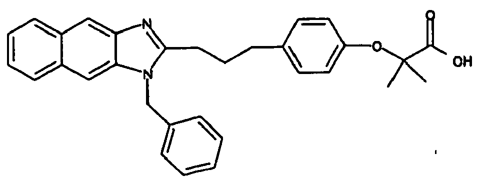

The ester from **Step A** (0.078 g, 0.00015 mol) is dissolved in ethanol and 2N NaOH is added. The reaction is refluxed for thirty minutes. Water is added to the mixture and the pH is adjusted to pH = 6. The desired acid precipitated out of solution. The material is filtered and dried (0.064 g, 86 %).
C₃₃H₃₀N₂O₃ (MW = 478.23); mass spectroscopy (MH⁺) = 479.3

### Example 6:

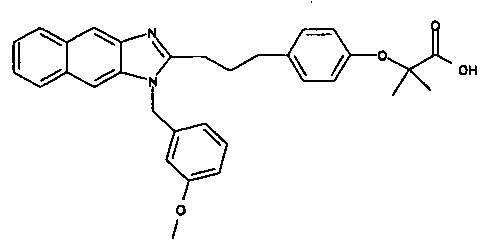

### Step A:

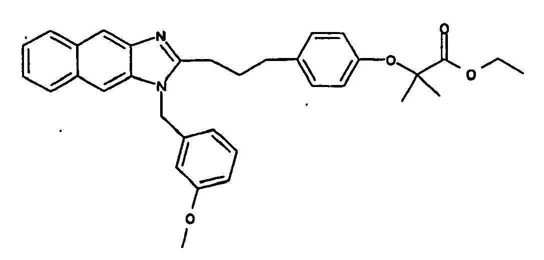

The ester from **Example 3, Step C** (0.243 g, 0.00058 mol) is dissolved in DMF (10 ml) and treated with CsCO₃ (0.475 g, 0.00146 mol) followed by 3-methoxy-benzyl bromide (0.129 ml, 0.00093 mol). The reaction is stirred for one hour at 65°C. Ether is added to the reaction and the layer is washed with water then brine. Purification by flash chromatography (4:1 hexanes: ethyl acetate) yields the ester (0.256 g, 83 %). C₃₄H₃₆N₂O₄ (MW = 536.27) ; mass spectroscopy (FD) = 536.4

### Step B:

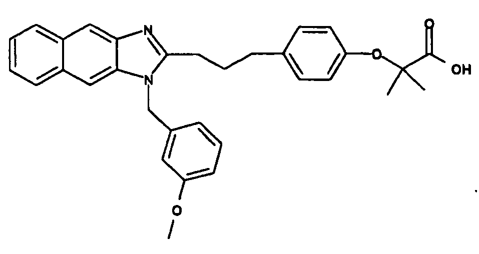

The ester from **Step A** (0.078 g, 0.00015 mol) is dissolved in ethanol (6 ml) and 2N NaOH (3 ml) is added. The reaction is refluxed for two and one-half hours. Water (30 ml) is added to the mixture and the pH is adjusted to pH = 6 using 1N HCl. The desired acid precipitated out of solution as yellow crystals. The material is filtered and dried (0.131 g, 65 %).
C₃₂H₃₂N₂O₄ (MW = 508.24); mass spectroscopy (MH⁺) = 509.4

### Example 7:

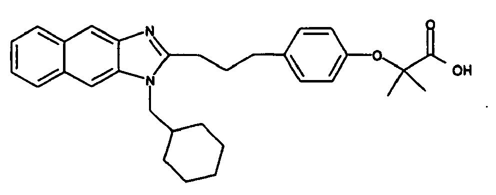

### Step A:

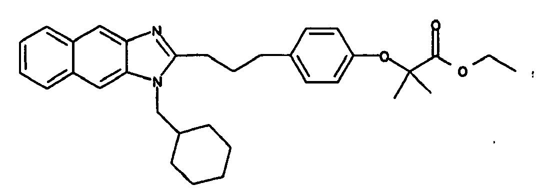

The ester from **Example 3, Step C** (0.122 g, 0.00029 mol) is dissolved in DMF and treated with CsCO₃ (0.238 g, 0.00073 mol) followed by bromomethyl cyclohexane (0.040 ml, 0.00029 mol). The reaction is stirred overnight at 65°C. Ether is added to the reaction and the layer is washed with water then brine. The product is carried forth without further purification (0.131 g, 88 %).
C₃₃H₄₀N₂O₃ (MW = 512.30) ; mass spectroscopy (MH⁺) = 513.1

### Step B:

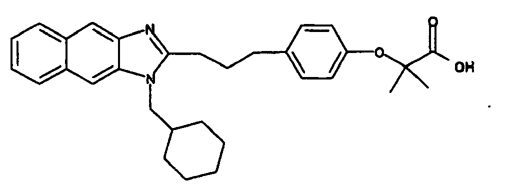

The ester from **Step A** (0.131 g, 0.00035 mol) is dissolved in ethanol (6 ml) and 2N NaOH (3 ml) is added. The reaction is refluxed. Water (35 ml) is added to the mixture and the pH is adjusted to pH = 6 using 1N HCl. The desired acid precipitated out of solution. Purification of the material by flash chromatography (100% methanol) yields the desired product.
C₃₁H₃₆N₂O₃ (MW = 484.27) ; mass spectroscopy (MH⁺) = 485.2

### Example 8:

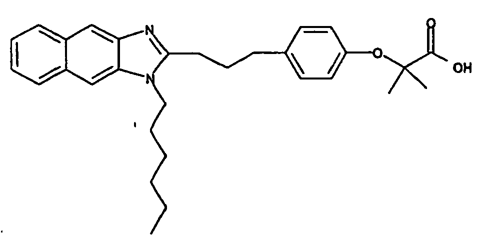

### Step A:

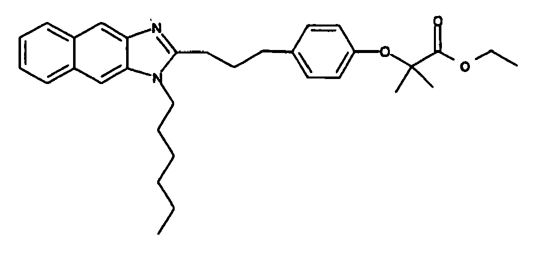

The ester from **Example 3, Step C** (0.117 g, 0.00028 mol) is dissolved in DMF and treated with CsCO₃ (0.228 g, 0.00070 mol) followed by 1-iodo-hexane (0.058 ml, 0.00028 mol). The reaction is stirred for four hours. Ether is added to the reaction and the layer is washed with water then brine. The product is carried forth without further purification (0.140 g, 100 %). C₃₂H₄₀N₂O₃ (MW = 500.30); mass spectroscopy (FD) = 500.5

### Step B:

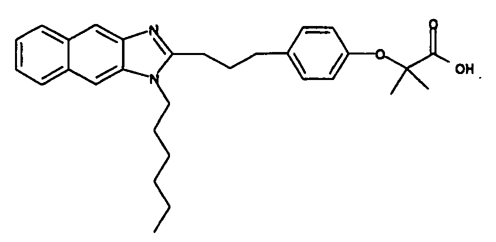

The ester from **Step A** (0.140 g, 0.00028 mol) is dissolved in ethanol and 2N NaOH is added. The reaction is refluxed. Water is added to the mixture and the pH is adjusted to pH = 6. The desired acid precipitated out of solution. The material is filtered and dried (0.047 g, 36 %).
C₃₀H₃₆N₂O₃ (MW = 472.27); mass spectroscopy (MH⁺) = 473.2

### Example 9:

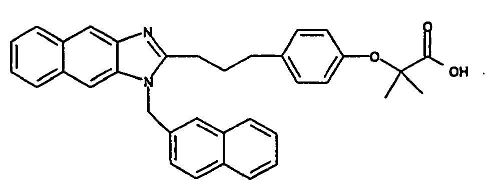

### Step A:

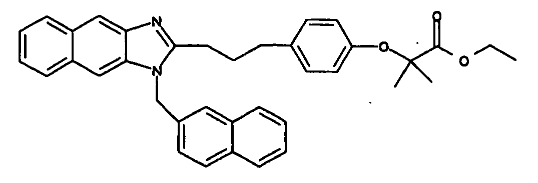

The ester from **Example 3, Step C** (0.500 g, 0.0012 mol) is dissolved in DMF (7 ml) and treated with CsCO₃ (0.975 g, 0.0030 mol) followed by 1-bromomethyl napthalene (0.398 ml, 0.0018 mol). The reaction is stirred overnight at 67°C. Ether is added to the reaction and the layer is washed with water. Purification by flash chromatography (3:1 hexanes: ethyl acetate; 1:1 hexanes: ethyl acetate) yields the alkylated ester (0.341 g, 51 %).
C₃₇H₃₆N₂O₃ (MW = 556.27); mass spectroscopy (MH⁺) = 557.3

### Step B:

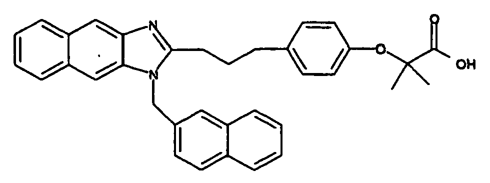

The ester from **Step A** (0.341 g, 0.00066 mol) is dissolved in ethanol and 2N NaOH is added. The reaction is refluxed for three hours. Water is added to the mixture and the pH is adjusted to pH = 7 using 1N HCl. The aqueous layer is extracted with ethyl acetate. The organic layer is concentrated to give the desired acid (0.040 g, 10%).
C₃₅H₃₂N₂O₃ (MW = 528.24); mass spectroscopy (MH⁺) = 529.2

### Example 10:

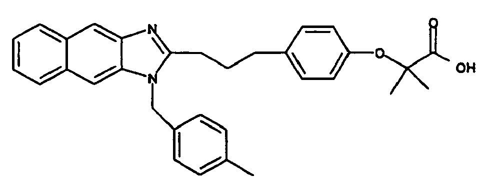

### Step A:

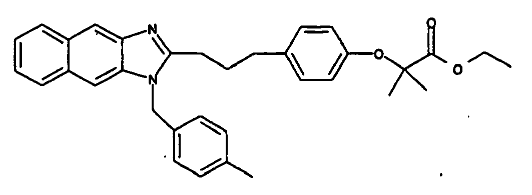

The ester from **Example 3, Step C** (0.300 g, 0.00072 mol) is dissolved in DMF and treated with CsCO₃ (0.585 g, 0.00180 mol) followed by 4-methyl benzyl bromide (0.200 g, 0.00108 mol). The reaction is stirred overnight at 67°C. Ether is added to the reaction and the layer is washed with water. Purification by flash chromatography (1:1 hexanes: ethyl acetate) yields the ester (0.261 g, 70 %).
C₃₄H₃₆N₂O₃ (MW = 520.27)

### Step B:

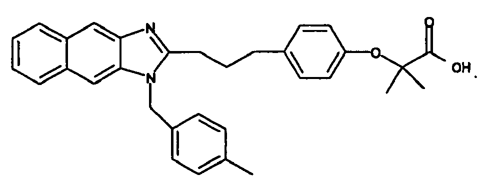

The ester from **Step A** (0.250 g, 0.00048 mol) is dissolved in ethanol and 2N NaOH is added. The reaction is refluxed for two hours. Water is added to the mixture and the pH is adjusted to pH = 6 using 1N HCl. The desired product precipitated out of solution. The precipitate is filtered and dried (0.217 g, 92 %).
C₃₂H₃₂N₂O₃ (MW = 492.62) ; mass spectroscopy (MH⁺) = 493.2

### Example 11:

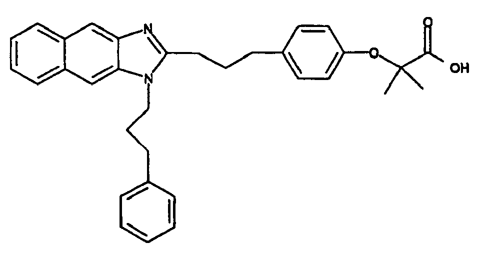

### Step A:

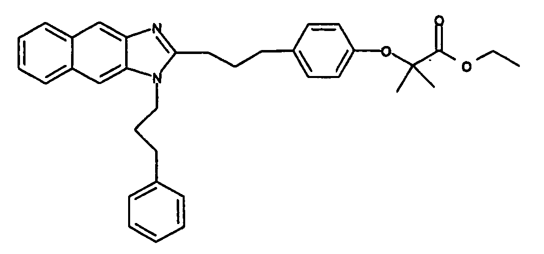

The ester from **Example 3, Step C** (0.500 g, 0.0012 mol) is dissolved in DMF (7 ml) and treated with CsCO₃ (0.975 g, 0.0030 mol) followed by 1-bromo-3-phenyl propane (0.274 ml, 0.0018 mol). The reaction is stirred overnight at 67°C. Ether is added to the reaction and the layer is washed with water. Purification by flash chromatography (3:1 hexanes: ethyl acetate; 1:1 hexanes: ethyl acetate) yields the alkylated ester (0.520 g, 81 %).
C₃₅H₃₈N₂O₃ (MW = 534.29); mass spectroscopy (MH⁺) = 535.3

### Step B:

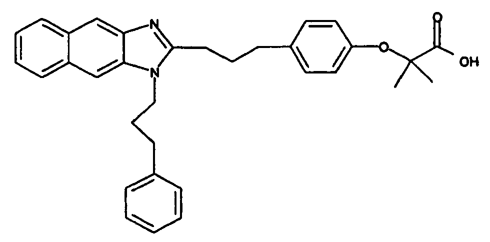

The ester from **Step A** (0.440 g, 0.00082 mol) is dissolved in ethanol (12 ml) and 2N NaOH (6 ml) is added. The reaction is refluxed for two hours. Water is added to the mixture and the pH is adjusted to pH = 7 using 1N HCl. The desired product precipitated out of solution. The product is filtered and dried product (0.137 g, 33 %).
C₃₃H₃₄N₂O₃ (MW = 506.26); mass spectroscopy (MH⁺) = 507.3

### Example 12:

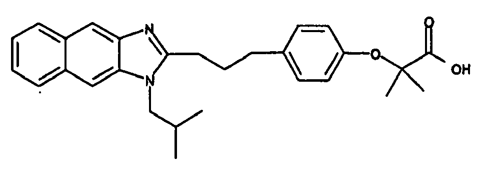

### Step A:

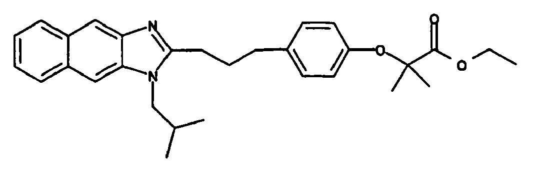

The ester from **Example 3, Step C** (0.500 g, 0.00120 mol) is dissolved in DMF (7 ml) and treated with CsCO₃ (0.975 g, 0.0030 mol) followed by 1-bromo-2-methyl-propane (0.196 ml, 0.0018 mol). The reaction is stirred overnight at 67°C. Ether is added to the reaction and the layer is washed with water. Purification by flash chromatography (1:1 hexanes ethyl acetate) yields the desired ester (0.355 g, 63 %). C₃₀H₃₆N₂O₃ (MW = 472.27); mass spectroscopy (MH⁺) = 473.3

### Step B:

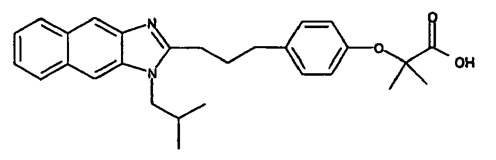

The ester from **Step A** (0.300 g, 0.00065 mol) is dissolved in methanol (7 ml) and treated with an aqueous solution of LiOH (0.18 M, 7 ml). The reaction is stirred overnight.
Water is added to the reaction mixture and the solution is extracted with ether. The aqueous layer is acidified to pH = 4 then extracted with ethyl acetate. The organic layer is concentrated to afford the desired acid (0.110 g, 38%). C₂₉H₃₂N₂O₃ (MW = 444.24); mass spectroscopy (MH⁺) = 445.2

### Example 13:

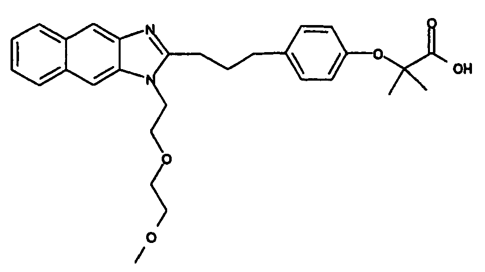

### Step A:

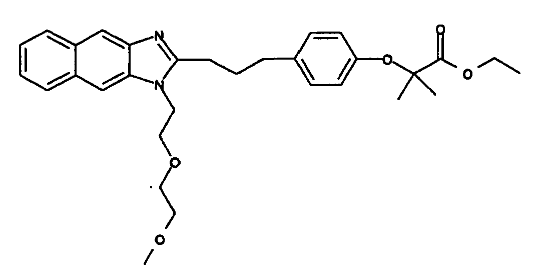

The ester from **Example 3, Step C** (0.500 g, 0.00120 mol) is dissolved in DMF and treated with CsCO₃ (1.27 g, 0.0039 mol) followed by (1-bromo-2-(2-methoxy-ethoxy) ethane (0.212 ml, 0.00156 mol). The reaction is stirred overnight at 65°C. Ether is added to the reaction and the layer is washed with water. Purification by flash chromatography (2:1 hexanes: ethyl acetate) yields the desired ester (0.723 g, 93 %). C₃₁H₃₈N₂O₅ (MW = 518.28)

### Step B:

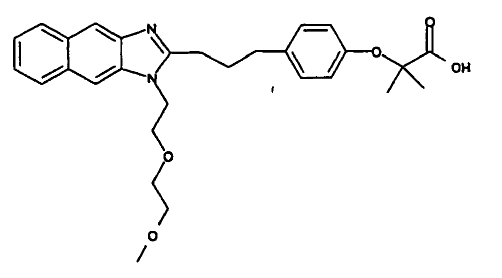

The ester from **Step A** (0.600 g, 0.00116 mol) is dissolved in methanol (7 ml) and treated with an aqueous solution of LiOH (0.33 M, 7 ml). The reaction is stirred overnight. Water is added to the reaction mixture and the solution is extracted with ether. The aqueous layer is acidified to pH = 4 then extracted with methylene chloride. The organic layer is concentrated to afford the desired acid (0.405 g, 71 %).
C₂₉H₃₄N₂O₅ (MW = 490.25) ; mass spectroscopy (MH⁺) = 491.1

### Example 14:

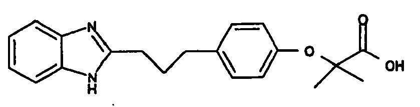

### Step A:

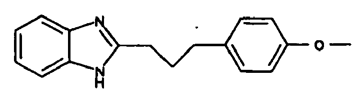

A THF solution of 4-(4-methoxyphenyl) butyric acid (10 g, 0.0515 mol) is cooled to -5°C and treated with triethyl amine (6.95 ml, 0.0499 mol) followed by isobutyl chloroformate (6.50 ml, 0.0497 mol). Phenylene diamine (5.95 g, 0.055 mol) is added and the reaction is stirred for four hours. The solvent is concentrated and the resulting residue is dissolved in ethyl acetate and extracted with water and 5% sodium bicarbonate then brine. Upon concentration of the organic layer, the material is dissolved in acetic acid (100 ml) and refluxed for two hours. The solvent is concentrated. Purification by flash chromatography (1:1 hexanes: ethyl acetate) afforded the desired product (5.87 g, 45%).
C₁₇H₁₈N₂O (MW = 266.14); mass spectroscopy (MH⁺) = 267.2

### Step B:

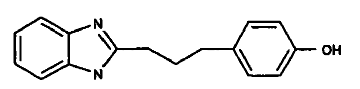

Boron tribromide (1.80 ml, 0.0194 mol) is added to methylene chloride and cooled to 0°C. The methyl ether from **Step A** (1.72 g, 0.0065 mol) is added. The reaction is stirred for one hour. A solution of 1:1 methylene chloride and methanol (14 mL) is added to quench the reaction. After stirring for some time, the solvent is concentrated. The crude residue is redissolved in ethyl acetate and washed with water. The desired material remained in the aqueous layer. The pH is adjusted to pH =7 upon which the product precipitated out of solution and is filtered (0.731 g, 44 %).
C₁₈H₂₀N₂O (MW = 2); mass spectroscopy (MH⁺) = 253.1

### Step C:

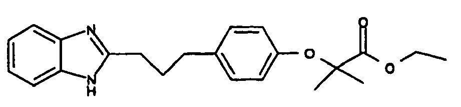

Phenol (5.5 g, 0.0210 mol) as described in **Step B** is dissolved in absolute ethanol (50 ml) and treated with K₂CO₃ (8.69 g, 0.0630 mol) followed by ethyl 2-bromoisobutyrate (22.0 ml, 0.153 mol). The reaction is stirred at 77°C. Upon cooling, the solvent is concentrated. The resulting residue is re-dissolved in methylene chloride and washed with water then brine. Purification by flash chromatography (4:1 hexanes: ethyl acetate;3:1 hexanes: ethyl acetate; 1:1 hexanes: ethyl acetate) yields the ester (3.62 g, 47%). C₂₂H₂₆N₂O₃ (MW = 366.19); mass spectroscopy (MH⁺) = 367.2

### Step D:

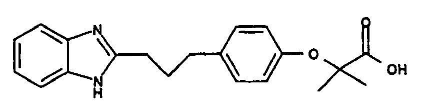

The ester from **Step B** (0.300 g, 0.00082 mol) is dissolved in methanol (3 ml) and treated with an aqueous solution of LiOH (0.55 M, 3 ml). The reaction is stirred overnight.
Water is added to the reaction mixture and the solution is extracted with ether. The aqueous layer is acidified to pH = 4 then extracted with methylene chloride. The organic layer is concentrated to afford the desired acid.
C₂₆H₂₆N₂O₃ (MW = 414.19) ; mass spectroscopy (MH⁺) = 415.2

### Example 15:

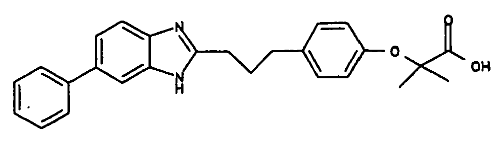

### Step A:

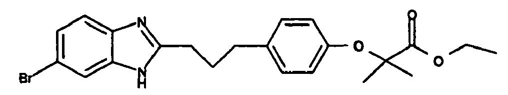

The ester from **Example 14 Step C** (3.13 g, 0.00860) is dissolved in methylene chloride. To the solution is added N-bromosuccinimide (1.52 g, 0.00860 mol) and silica gel (3.00 g). The reaction is stirred overnight. The silica gel is filtered and washed with methanol. The filtrate is concentrated and the resulting material is dissolved in methylene chloride and extracted with water. The desired product is obtained and carried forth without further purification (3.5 g, 90%).
C₂₂H₂₅N₂O₃Br (MW = 444.10)

### Step B:

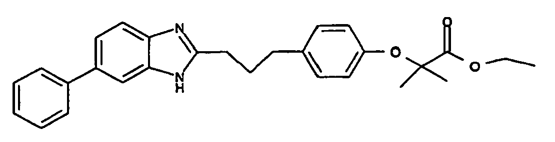

The substrate from **Step A** (0.500 g, 0.0011 mol) is combined with phenyl boronic acid (0.134 g, 0.0011 mol) and potassium carbonate (0.151 g, 0.0011 mol) in a solution of dioxane and water (4:1). The solution is de-oxygenated. Tetrakis (triphenyl phospine) palladium (0) is added and the mixture is stirred at 90°C. Upon concentration of the solvent, the residue is re- dissolved in methylene chloride and washed with water and brine. Purification by flash chromatography (2:1 hexanes: ethyl acetate) yields the desired product. C₂₈H₃₀N₂O₃ (MW = 442.23); mass spectroscopy (MH⁺) = 443.0

### Step C:

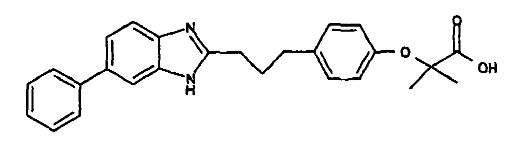

The ester from **Step B** (0.193 g, 0.00040 mol) is dissolved in methanol (2 ml) and treated with an aqueous solution of LiOH (0.44 M, 2 ml). The reaction is stirred overnight.
Water is added to the reaction mixture and the solution is extracted with ether. The aqueous layer is acidified to pH = 4 then extracted with methylene chloride. The organic layer is concentrated to afford the desired acid as a white solid. C₂₆H₂₆N₂O₃ (MW = 414.19); mass spectroscopy (MH⁺) = 415.2

### Example 16:

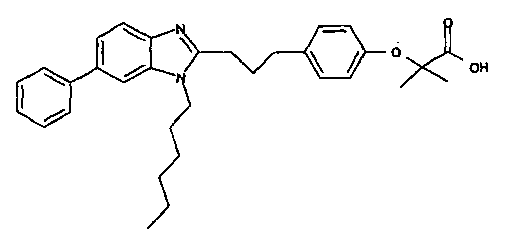

### Step A:

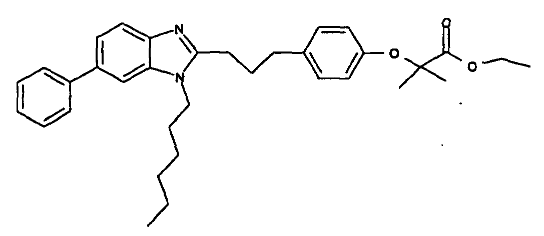

The ester from **Example 15, Step B** (0.372 g, 0.00084 mol) is dissolved in DMF and treated with CsCO₃ (0.683 g, 0.00210 mol) followed by 1-iodo-hexane (0.186 ml, 0.00126 mol). The reaction is stirred at 67°C. Ether is added to the reaction and the layer is washed with water then brine. Purification by flash chromatography (2:1 hexanes: ethyl acetate) yields the desired product (0.172 g, 50 %).
C₃₄H₄₂N₂O₃ (MW = 526.32) ; mass spectroscopy (MH⁺) = 527.3

### Step C:

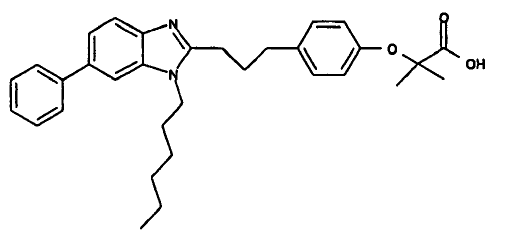

The ester from **Step B** (0.160 g, 0.00030 mol) is dissolved in methanol (2 ml) and treated with an aqueous solution of LiOH (0.3 M, 2 ml). The reaction is stirred overnight.
Water is added to the reaction mixture and the solution is extracted with ether. The aqueous layer is acidified to pH = 4 then extracted with methylene chloride. The organic layer is concentrated to afford the desired acid as a white solid.
C₃₂H₃₈N₂O₃ (MW = 498.29) ; mass spectroscopy (MH⁺) = 499.3

### Biological Assays

### Binding and Cotransfection Studies

The in vitro potency of compounds in modulating PPARα receptors are determined by the procedures detailed below. DNA-dependent binding (ABCD binding) is carried out using SPA technology with PPAR receptors. Tritium-labeled PPARα agonists are used as radioligands for generating displacement curves and IC₅₀ values with compounds of the invention. Cotransfection assays are carried out in CV-1 cells. The reporter plasmid contained an acylCoA oxidase (AOX) PPRE and TK promoter upstream of the luciferase reporter cDNA. Appropriate PPARs are constitutively expressed using plasmids containing the CMV promoter. For PPARα, interference by endogenous PPARγ in CV-1 cells is an issue. In order to eliminate such interference, a GAL4 chimeric system is used in which the DNA binding domain of the transfected PPAR is replaced by that of GAL4, and the GAL4 response element is utilized in place of the AOX PPRE. Cotransfection efficacy is determined relative to PPARα agonist reference molecules. Efficacies are determined by computer fit to a concentration-response curve, or in some cases at a single high concentration of agonist (10 µM).

These studies are carried out to evaluate the ability of compounds of the invention to bind to and/or activate various nuclear transcription factors, particularly huPPARα ("hu" indicates "human"). These studies provide in vitro data concerning efficacy and selectivity of compounds of the invention. Furthermore, binding and cotransfection data for compounds of the invention are compared with corresponding data for marketed compounds that act on huPPARα.

The binding and cotransfection efficacy values for compounds of the invention which are especially useful for modulating a PPAR receptor, are ≤ 100 nM and ≥ 50%, respectively.

### Evaluation of Triglyceride Reduction and HDL Cholesterol Elevation in HuapoAI Transgenic Mice

Compounds of the present invention are studied for effects upon HDL and triglyceride levels in human apoAI mice. For each compound tested, seven to eight week old male mice, transgenic for human apoAI (C57BL/6-tgn(apoal)lrub, Jackson Laboratory, Bar Harbor, ME) are acclimated in individual cages for two weeks with standard chow diet (Purina 5001) and water provided ad libitum.
After the acclimation, mice and chow are weighed and assigned to test groups (n = 5) with randomization by body weight. Mice are dosed daily by oral gavage for 8 days using a 29 gauge, 1-1/2 inch curved feeding needle (Popper & Sons). The vehicle for the controls, test compounds and the positive control (fenofibrate 100mg/kg) is 1% carboxymethylcellulose (w/v) with 0.25% tween 80 (w/v). All mice are dosed daily between 6 and 8 a.m. with a dosing volume of 0.2ml. Prior to termination, animals and diets are weighed and body weight change and food consumption are calculated. Three hours after last dose, mice are euthanized with CO2 and blood is removed (0.5-1.0 ml) by cardiac puncture. After sacrifice, the liver, heart, and epididymal fat pad are excised and weighed. Blood is permitted to clot and serum is separated from the blood by centrifugation.

Cholesterol and triglycerides are measured colorimetrically using commercially prepared reagents (for example, as available from Sigma #339-1000 and Roche #450061 for triglycerides and cholesterol, respectively). The procedures are modified from published work (McGowan M. W. et al., Clin Chem 29:538-542,1983; Allain C. C. et al., Clin Chem 20:470-475,1974. Commercially available standards for triglycerides and total cholesterol, respectively, commercial quality control plasma, and samples are measured in duplicate using 200 µl of reagent. An additional aliquot of sample, added to a well containing 200 µl water, provided a blank for each specimen. Plates are incubated at room temperature on a plate shaker and absorbance is read at 500 nm and 540 nm for total cholesterol and triglycerides, respectively. Values for the positive control are always within the expected range and the coefficient of variation for samples is below 10%. All samples from an experiment are assayed at the same time to minimize inter-assay variability.

Serum lipoproteins are separated and cholesterol quantitated by fast protein liquid chromatography (FPLC) coupled to an in line detection system. Samples are applied to a Superose 6 HR size exclusion column (Amersham Pharmacia Biotech) and eluted with phosphate buffered saline-EDTA at 0.5 ml/min. Cholesterol reagent (Roche Diagnostics Chol/HP 704036) at 0.16ml/min mixed with the column effluent through a T-connection and the mixture passed through a 15 m x 0.5 mm id knitted tubing reactor immersed in a 37 C water bath. The colored product produced in the presence of cholesterol is monitored in the flow strem at 505 nm and the analog voltage from the monitor is converted to a digital signal for collection and analysis. The change in voltage corresponding to change in cholesterol concentration is plotted vs time and the area under the curve corresponding to the elution of very low density lipoprotein (VLDL), low density lipoprotein (LDL) and high density lipoprotein (HDL) is calculated using Perkin Elmer Turbochrome software.

Triglyceride Serum Levels in Mice Dosed with a Compound of the Invention is Compared to Mice Receiving the Vehicle to identify compounds which could be particularly useful for lowering triglycerides. Generally, triglyceride decreases of greater than or equal to 30% (thirty percent) compared to control following a 30 mg/kg dose suggests a compound that can be especially useful for lowering triglyceride levels.

The percent increase of HDLc serum levels in mice receiving a compound of the invention is compared to mice receiving vehicle to identify compounds of the invention that could be particularly useful for elevating HDL levels. Generally, and increase of greater than or equal to 25% (twenty five percent) increase in HDLc level following a 30 mg/kg dose suggests a compound that can be especially useful for elevating HDLc levels.

It may be particularly desirable to select compounds of this invention that both lower triglyceride levels and increase HDLc levels. However, compounds that either lower triglyceride levels or increase HDLc levels may be desirable as well.

### Evaluation of Glucose Levels in db/db Mice

The effects upon plasma glucose associated with administering various dose levels of different compounds of the present invention and the PPAR gamma agonist rosiglitazone (BRL49653) or the PPAR alpha agonist fenofibrate, and the control, to male db/db mice, are studied.

Five week old male diabetic (db/db) mice [for example, C57BlKs/j-m +/+ Lepr(db), Jackson Laboratory, Bar Harbor, ME] or lean littermates are housed 6 per cage with food and water available at all times. After an acclimation period of 2 weeks, animals are individually identified by ear notches, weighed, and bled via the tail vein for determination of initial glucose levels. Blood is collected (100 µl) from unfasted animals by wrapping each mouse in a towel, cutting the tip of the tail with a scalpel, and milking blood from the tail into a heparinized capillary tube. Sample is discharged into a heparinized microtainer with gel separator and retained on ice. Plasma is obtained after centrifugation at 4°C and glucose measured immediately. Remaining plasma is frozen until the completion of the experiment, when glucose and triglycerides are assayed in all samples. Animals are grouped based on initial glucose levels and body weights. Beginning the following morning, mice are dosed daily by oral gavage for 7 days. Treatments are test compounds (30 mg/kg), a positive control agent (30 mg/kg) or vehicle [1% carboxymethylcellulose (w/v)/ 0.25% Tween80 (w/v); 0.3 ml/mouse]. On day 7, mice are weighed and bled (tail vein) 3 hours after dosing. Twenty-four hours after the 7^{th} dose (i.e., day 8), animals are bled again (tail vein). Samples obtained from conscious animals on days 0, 7 and 8 are assayed for glucose. After the 24-hour bleed, animals are weighed and dosed for the final time. Three hours after dosing on day 8, animals are anesthetized by inhalation of isoflurane and blood obtained via cardiac puncture (0.5-0.7 ml). Whole blood is transferred to serum separator tubes, chilled on ice and permitted to clot. Serum is obtained after centrifugation at 4°C and frozen until analysis for compound levels. After sacrifice by cervical dislocation, the liver, heart and epididymal fat pads are excised and weighed.

Glucose is measured colorimetrically using commercially purchased reagents. According to the manufacturers, the procedures are modified from published work (McGowan, M. W., Artiss, J. D., Strandbergh, D. R. & Zak, B. Clin Chem, 20:470-5 (1974) and Keston, A. Specific colorimetric enzymatic analytical reagents for glucose. Abstract of papers 129th Meeting ACS, 31C (1956).); and depend on the release of a mole of hydrogen peroxide for each mole of analyte, coupled with a color reaction first described by Trinder (Trinder, P. Determination of glucose in blood using glucose oxidase with an alternative oxygen acceptor. Ann Clin Biochem, 6:24 (1969)). The absorbance of the dye produced is linearly related to the analyte in the sample. The assays are further modified in our laboratory for use in a 96 well format. The commercially available standard for glucose, commercially available quality control plasma, and samples (2 or 5 µl/well) are measured in duplicate using 200 µl of reagent. An additional aliquot of sample, pipetted to a third well and diluted in 200 µl water, provided a blank for each specimen. Plates are incubated at room temperature for 18 minutes for glucose on a plate shaker (DPC Micormix 5) and absorbance read at 500 nm on a plate reader. Sample absorbances are compared to a standard curve (100-800 for glucose). Values for the quality control sample are always within the expected range and the coefficient of variation for samples is below 10%. All samples from an experiment are assayed at the same time to minimize inter-assay variability.

### Evaluation of the Effects of Compounds of the Present Invention upon A^{y} Mice Body Weight, Fat Mass, Glucose and Insulin Levels

### Female A^{y} Mice

Female A^{y} mice are singly housed, maintained under standardized conditions (22°C, 12 h light:dark cycle), and provided free access to food and water throughout the duration of the study. At twenty weeks of age the mice are randomly assigned to vehicle control and treated groups based on body weight and body fat content as assessed by DEXA scanning (N=6). Mice are then dosed via oral gavage with either vehicle or a Compound of this invention (50 mg/kg) one hour after the initiation of the light cycle (for example, about 7 A.M.) for 18 days. Body weights are measured daily throughout the study. On day 14 mice are maintained in individual metabolic chambers for indirect calorimetry assessment of energy expenditure and fuel utilization. On day 18 mice are again subjected to DEXA scanning for post treatment measurement of body composition.

The results of p.o. dosing of compound for 18 days on body weight, fat mass, and lean mass are evaluated and suggest which compounds of this invention can be especially useful for maintaining desirable weight and/or promoting desired lean to fat mass.

Indirect calorimetry measurements revealing a significant reduction in respiratory quotient (RQ) in treated animals during the dark cycle [0.864 ± 0.013 (Control) vs. 0.803 ± 0.007 (Treated); p < 0.001] is indicative of an increased utilization of fat during the animals' active (dark) cycle and can be used to selected especially desired compounds of this invention. Additionally, treated animals displaying significantly higher rates of energy expenditure than control animals suggest such compounds of this invention can be especially desired.

### Male KK/A^{y} Mice

Male KK/A^{y} mice are singly housed, maintained under standardized conditions (22°C, 12 h light:dark cycle), and provided free access to food and water throughout the duration of the study. At twenty-two weeks of age the mice are randomly assigned to vehicle control and treated groups based on plasma glucose levels. Mice are then dosed via oral gavage with either vehicle or a Compound of this invention (30 mg/kg) one hour after the initiation of the light cycle (7 A.M.) for 14 days. Plasma glucose, triglyceride, and insulin levels are assessed on day 14.

The results of p.o. dosing of compound for 14 days on plasma glucose, triglycerides, and insulin are evaluated to identify compounds of this invention which may be especially desired.

### Method to Elucidate the LDL-cholesterol Total-cholesterol and Triglyceride Lowering Effect

Male Syrian hamsters (Harlan Sprague Dawley) weighing 80-120 g are placed on a high-fat cholesterol-rich diet for two to three weeks prior to use. Feed and water are provided ad libitum throughout the course of the experiment. Under these conditions, hamsters become hypercholesterolemic showing plasma cholesterol levels between 180-280 mg/dl. (Hamsters fed with normal chow have a total plasma cholesterol level between 100-150 mg/dl.) Hamsters with high plasma cholesterol (180 mg/dl and above) are randomized into treatment groups based on their total cholesterol level using the GroupOptimizeV211.xls program.

A Compound of this invention is dissolved in an aqueous vehicle (containing CMC with Tween 80) such that each hamster received once a day approx. 1 ml of the solution by garvage at doses 3 and 30 mg/kg body weight. Fenofibrate (Sigma Chemical, prepared as a suspension in the same vehicle) is given as a known alpha-agonist control at a dose of 200 mg/kg, and the blank control is vehicle alone. Dosing is performed daily in the early morning for 14 days.

Quantification of Plasma Lipids :
On the last day of the test, hamsters are bled (400 ul) from the suborbital sinus while under isoflurane anesthesia 2 h after dosing. Blood samples are collected into heparinized microfuge tubes chilled in ice bath. Plasma samples are separated from the blood cells by brief centrifugation. Total cholesterol and triglycerides are determined by means of enzymatic assays carried out automatically in the Monarch equipment (Instrumentation Laboratory) following the manufacturer's precedure. Plasma lipoproteins (VLDL, LDL and HDL) are resolved by injecting 25 ul of the pooled plasma samples into an FPLC system eluted with phosphate buffered saline at 0.5 ml/min through a Superose 6 HR 10/30 column (Pharmacia) maintained room temp. Detection and characterization of the isolated plasma lipids are accomplished by postcolumn incubation of the effluent with a Cholesterol/HP reagent (for example, Roche Lab System; infused at 0.12 ml/min) in a knitted reaction coil maintained at 37°C. The intensity of the color formed is proportional to the cholesterol concentration and is measured photometrically at 505 nm.

The effect of administration of a Compound of this invention for 14 days is studied for the percent reduction in LDL level with reference to the vehicle group.
Especially desired compounds are markedly more potent than fenofibrate in LDL-lowering efficacy. Compounds of this invention that decrease LDL greater than or equal to 30% (thirty percent) compared to vehicle can be especially desired.

The total-cholesterol and triglyceride lowering effects of a Compound of this invention is also studied. The data for reduction in total cholesterol and triglyceride levels after treatment with a compound of this invention for 14 days is compared to the vehicle to suggest compounds that can be particularly desired. The known control fenofibrate did not show significant efficacy under the same experimental conditions.

### Method to Elucidate the Fibrinogen-Lowering Effect of PPAR Modulators

### Zucker Fatty Rat Model:

The life phase of the study on fibrinogen-lowering effect of compounds of this invention is part of the life phase procedures for the antidiabetic studies of the same compounds. On the last (14^{th}) day of the treatment period, with the animals placed under surgical anesthesia, - 3ml of blood is collected, by cardiac puncture, into a syringe containing citrate buffer. The blood sample is chilled and centrifuged at 4°C to isolate the plasma that is stored at - 70 °C prior to fibrinogen assay.

### Quantification of Rat Plasma Fibrinogen:

Rat plasma fibrinogen levels are quantified by using a commercial assay system consists of a coagulation instrument following the manufacturer's protocol. In essence, 100 ul of plasma is sampled from each specimen and a 1/20 dilution is prepared with buffer. The diluted plasma is incubated at 37°C for 240 seconds. Fifty microliters of clotting reagent thrombin solution (provided by the instrument's manufacturer in a standard concentration) is then added. The instrument monitors the clotting time, a function of fibrinogen concentration quantified with reference to standard samples. Compounds that lower fibrinogen level greater than vehicle can be especially desired.

Cholesterol and triglyceride lowering effects of compounds of this invention are also studied in Zucker rats.

### Method to Elucidate the Anti-body Weight Gain and Anti-appetite Effects of Compounds of this invention

### Fourteen-Day Study in Zucker Fatty Rat¹ or ZDF Rat² Models :

Male Zucker Fatty rats, non-diabetic (Charles River Laboratories, Wilmington, MA) or male ZDF rats (Genetic Models, Inc, Indianapolis, IN) of comparable age and weight are acclimated for 1 week prior to treatment. Rats are on normal chow and water is provided ad libitum throughout the course of the experiment.

Compounds of this invention are dissolved in an aqueous vehicle such that each rat received once a day approximately 1 ml of the solution by garvage at doses 0.1, 0.3, 1 and 3 mg/kg body weight. Fenofibrate (Sigma Chemical, prepared as a suspension in the same vehicle) a known alpha-agonist given at doses of 300 mg/kg, as well as the vehicle are controls. Dosing is performed daily in the early morning for 14 days. Over the course of the experiment, body weight and food consumption are monitored.
Using this assay, compounds of this invention are identified to determine which can be associated with a significant weight reduction.

### EQUIVALENTS:

While this invention has been particularly shown and described with references to preferred embodiments thereof, it will be understood by those skilled in the art that various changes in form and details may be made therein without departing from the scope of the invention encompassed by the appended claims.

## Claims

1. A compound of Formula I: 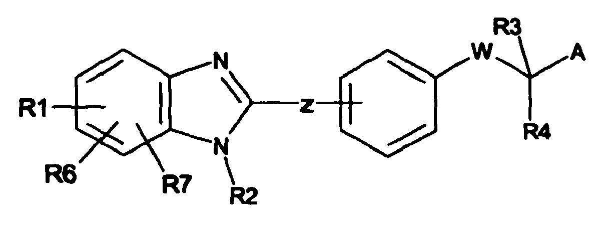 and pharmaceutically acceptable salts thereof, wherein:
(a) R1 is selected from the group consisting of hydrogen, C₁-C₈ alkyl, aryl-C₀₋₄-alkyl, heteroaryl-C₀₋₄-alkyl, and C3-C6 cycloalkylaryl-C₀₋₂-alkyl, wherein said C₁-C₈ alkyl, aryl-C₀₋₄-alkyl, heteroaryl-C₀₋₄-alkyl, and C3-C6 cycloalkylaryl-C₀₋₂-alkyl is each optionally substituted with from one to three substituents each independently selected from R1';
(b) R1', R2', R4', R6', A', Z' and R19' are each the group consisting of C₁-C₅ alkyl, C₁-C₅ alkoxy, C₁-C₅ haloalkyl, C₁-C₅ haloalkoxy, nitro, cyano, CHO, hydroxyl, C₁-C₄ alkanoic acid phenyl, aryloxy, SO₂R16, SR5, benzyloxy, alkylcarboxamido and COOH;
(c) R2 is selected from the group consisting of hydrogen, (C₂-C₄) alkyl-O- (C₂-C₄) alkyl-O- (C₁-C₄) alkyl, C₁-C₈ alkylene, aryl-C₀₋₄-alkyl, heteroaryl-C₀₋₄-alkyl, and C₃-C₆ cycloalkyl-C₀₋₄-alkyl, and wherein said (C₂-C₄)alkyl-O-(C₂-C₄)alkyl-O-(C₁-C₄) alkyl, C₁-C₈ alkylene, aryl-C₀₋₄-alkyl, heteroaryl-C₀₋₄-alkyl, and C₃-C₆ cycloalkyl-C₀₋₄-alkyl, is each optionally substituted with from one to three substituents each independently selected from R2';
(d) R3 is selected from the group consisting of hydrogen, C₁-C₅ alkyl, and C₁-C₅ alkoxy;
(e) R4 is selected from the group consisting of hydrogen, C₁-C₅ alkyl, C₁-C₅ alkoxy, C₃-C₆ cycloalkyl, and aryl C₀-C₄ alkyl, and wherein said C₁-C₅ alkyl, C₁-C₅ alkoxy, C₃-C₆ cycloalkyl, and aryl C₀-C₄ alkyl is each optionally substituted with from one to three substituents each independently selected from R4'; and wherein R3 and R4 are optionally combined to form a C₃-C₄ cycloalkyl;
(f) R5 and R16 are each selected from the group consisting of hydrogen, (C₁-C₆) alkyl and halo (C₁-C₆)alky;
(g) R6 and R7 are each independently selected from the group consisting of hydrogen, (C₁-C₆) alkyl, (C₁-C₆) alkenyl, halo (C₁-C₆) alkyl, halo, oxy, (C₁-C₆) alkoxy, and wherein said (C₁-C₆) alkyl, halo (C₁-C₆) alkyl, and (C₁-C₆) alkoxy are each is each optionally substituted with from one to three substituents each independently selected from R6'; and wherein R6 and R7 optionally combine to form a C3-C6 aryl that is fused to the group from which R6 and R7 each originate;
(h) W is selected from the group consisting of O, C, N and S;
(i) Z is C₃ alkyl optionally substituted with Z';
(j) A is selected from the group consisting of carboxyl, carboxamide, sulfonamide, acylsulfonamide, tetrazole, and (CH₂)ₙ COOR19, and wherein said sulfonamide, acylsulfonamide, and tetrazole is each optionally substituted with from one to three substituents each independently selected from A';
(k) n is 0, 1, 2 or 3; and
(l) R19 is selected from the group consisting of hydrogen, C1-C4alkyl and arylmethyl, wherein said alkyl and arylmethyl is each optionally substituted with from one to three substituents each independently selected from R19'.

2. A compound as claimed by Claims 1 wherein W is O.

3. A compound as claimed by any one of Claims 1 or 2 wherein A is COOH.

4. A compound as claimed by any one of Claims 1, 2, or 3 wherein Z is unsubstituted.

5. A compound as claimed by any one of Claims 1, 2, 3, or 4 wherein R6 and R7 are each C1-C2 alkyl.

6. A compound as claimed by any one of Claims 1, 2, 3 or 4 wherein R6 and R7 combine to form a fused 6 member cyclic aromatic.

7. A compound as claimed by any one of Claims 1, 2, 3 or 4 wherein R1 is phenyl.

8. A compound as claimed by any one of Claims 1, 2, 3, 4, 5, 6, or 7 wherein R2 is straight or branched C₁-C₆ alkyl.

9. A compound as claimed by any one of Claims 1, 2, 3, 4, 5, 6, or 7 wherein R2 is (C₁-C₃)alkyl-phenyl or (C₁-C₃)alkyl-naphthyl.

10. A compound of Claim 9 wherein the phenyl or naphthyl is substituted with one or two substituents independently selected from the group consisting of C₁-C₃ alkyl, halo, and C₁-C₃ alkoxy.

11. A pharmaceutical composition, comprising a pharmaceutically acceptable carrier and at least one compound as claimed by any one of Claims 1 to 10.

12. Use of a compound of any one of Claims 1 to 10 for the manufacture of a medicament for the treatment of a condition modulated by a peroxisome proliferator activated receptor.

13. Use according to Claim 12 wherein the peroxisome proliferator activated receptor is selectively modulated PPAR α.

14. Use of a compound of any one of Claims 1 to 10 for the manufacture of a medicament for the treatment or prevention of diabetes mellitus in a mammal.

15. Use of a compound of any one of Claims 1 to 10 for the manufacture of a medicament for the treatment of Syndrome X in a mammal.

16. Use of a compound of any one of Claims 1 to 10 for the manufacture of a medicament for the treatment or prevention of cardiovascular disease in a mammal.

17. Use according to Claim 16 wherein the cardiovascular disease is atherosclerosis.

## Patentansprüche

1. Verbindung der Formel I 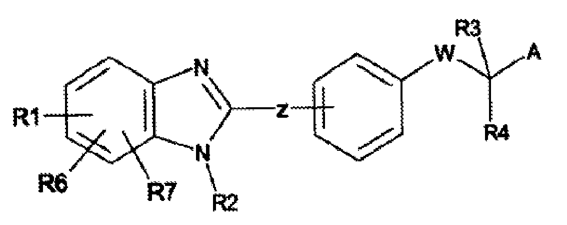 und pharmazeutisch akzeptable Salze hiervon, worin
(a) R1 aus der Gruppe ausgewählt ist, die besteht aus Wasserstoff, C₁-C₈-Alkyl, Aryl-C₀-C₄-alkyl, Heteroaryl-C₀-C₄-alkyl und C₃-C₆-Cycloalkylaryl-C₀-C₂-alkyl, worin diese Gruppen C₁-C₈-Alkyl, Aryl-C₀-C₄-alkyl, Heteroaryl-C₀-C₄-alkyl und C₃-C₆-Cycloalkylaryl-C₀-C₂-alkyl jeweils optional substituiert sind durch ein bis drei Substituenten, die unabhängig ausgewählt sind aus R1',
(b) R1', R2', R4', R6', A', Z' und R19' jeweils Gruppen sind, die bestehen aus C₁-C₅-Alkyl, C₁-C₅-Alkoxy, C₁-C₅-Halogenalkyl, C₁-C₅-Halogenalkoxy, Nitro, Cyano, CHO, Hydroxyl, C₁-C₄-Alkancarbonsäurephenyl, Aryloxy, SO₂R16, SR5, Benzyloxy, Alkylcarboxamid und COOH,
(c) R2 aus der Gruppe ausgewählt ist, die besteht aus Wasserstoff, (C₂-C₄)-Alkyl-O-(C₂-C₄)-alkyl-O-(C₁-C₄)-alkyl, C₁-C₈-Alkylen, Aryl-C₀-C₄-alkyl, Heteroaryl-C₀-C₄-alkyl und C₃-C₆-Cycloalkyl-C₀-C₄-alkyl, und worin diese Gruppen (C₂-C₄)-Alkyl-O-(C₂-C₄)-alkyl-O-(C₁-C₄)-alkyl, C₁-C₈-Alkylen, Aryl-C₀-C₄-alkyl, Heteroaryl-C₀-C₄-alkyl und C₃-C₆-Cycloalkyl-C₀-C₄-alkyl jeweils optional substituiert sind mit ein bis drei Substituenten, die unabhängig ausgewählt sind aus R2',
(d) R3 aus der Gruppe ausgewählt ist, die besteht aus Wasserstoff, C₁-C₅-Alkyl und C₁-C₅-Alkoxy,
(e) R4 aus der Gruppe ausgewählt ist, die besteht aus Wasserstoff, C₁-C₅-Alkyl, C₁-C₅-Alkoxy, C₃-C₆-Cycloalkyl und Aryl-C₀-C₄-alkyl, wobei diese Gruppen C₁-C₅-Alkyl, C₁-C₅-Alkoxy, C₃-C₆-Cycloalkyl und Aryl-C₀-C₄-alkyl jeweils optional substituiert sind durch ein bis drei Substituenten, die unabhängig ausgewählt sind aus R4', und worin R3 und R4 optional zusammen ein C₃-C₄-Cycloalkyl bilden,
(f) R5 und R16 jeweils aus der Gruppe ausgewählt sind, die besteht aus Wasserstoff, (C₁-C₆)-Alkyl und Halogen-(C₁-C₆)-alkyl,
(g) R6 und R7 jeweils unabhängig aus der Gruppe ausgewählt sind, die besteht aus Wasserstoff, (C₁-C₆)-Alkyl, (C₁-C₆)-Alkenyl, Halogen-(C₁-C₆)-alkyl, Halogen, Oxy und (C₁-C₆)-Alkoxy, wobei diese Gruppen (C₁-C₆)-Alkyl, Halogen-(C₁-C₆)-alkyl und (C₁-C₆)-Alkoxy jeweils optional substituiert sind durch ein bis drei Substituenten, die jeweils unabhängig ausgewählt sind aus R6', und worin R6 und R7 optional zusammen eine Gruppe C₃-C₆-Aryl bilden, die an die Gruppe fusioniert ist, welche jeweils aus R6 und R7 entspringt,
(h) W aus der Gruppe ausgewählt ist, die besteht aus O, C, N und S,
(i) Z für C₃-Alkyl steht, das optional substituiert ist durch Z',
(j) A aus der Gruppe ausgewählt ist, die besteht aus Carboxyl, Carboxamid, Sulfonamid, Acylsulfonamid, Tetrazol und (CH₂)ₙ-COOR19, worin diese Gruppen Sulfonamid, Acylsulfonamid und Tetrazol jeweils optional substituiert sind durch ein bis drei Substituenten, die jeweils unabhängig ausgewählt sind aus A',
(k) n für 0, 1, 2 oder 3 steht, und
(l) R19 aus der Gruppe ausgewählt ist, die besteht aus Wasserstoff, C₁-C₄-Alkyl und Arylmethyl, worin diese Gruppen Alkyl und Arylmethyl jeweils optional substituiert sind durch ein bis drei Substituenten, die jeweils unabhängig ausgewählt sind aus R19'.

2. Verbindung nach Anspruch 1, worin W für O steht.

3. Verbindung nach einem der Ansprüche 1 oder 2, worin A für COOH steht.

4. Verbindung nach einem der Ansprüche 1, 2 oder 3, worin Z unsubstituiert ist.

5. Verbindung nach einem der Ansprüche 1, 2, 3 oder 4, worin R6 und R7 jeweils für C₁-C₂-Alkyl stehen.

6. Verbindung nach einem der Ansprüche 1, 2, 3 oder 4, worin R6 und R7 zusammen einen fusionierten 6-gliedrigen cycloaromatischen Ring bilden.

7. Verbindung nach einem der Ansprüche 1, 2, 3 oder 4, worin R1 für Phenyl steht.

8. Verbindung nach einem der Ansprüche 1, 2, 3, 4, 5, 6 oder 7, worin R2 für geradkettiges oder verzweigtkettiges C₁-C₆-Alkyl steht.

9. Verbindung nach einem der Ansprüche 1, 2, 3, 4, 5, 6 oder 7, worin R2 für (C₁-C₃)-Alkylphenyl oder (C₁-C₃)-Alkylnaphthyl steht.

10. Verbindung nach Anspruch 9, worin das Phenyl oder Naphthyl durch ein oder zwei Substituenten substituiert ist, die unabhängig ausgewählt sind aus der Gruppe, die besteht aus C₁-C₃-Alkyl, Halogen und C₁-C₃-Alkoxy.

11. Pharmazeutische Zusammensetzung, die umfasst einen pharmazeutisch akzeptablen Träger und wenigstens eine Verbindung nach einem der Ansprüche 1 bis 10.

12. Verwendung einer Verbindung nach einem der Ansprüche 1 bis 10 zur Herstellung eines Arzneimittels für die Behandlung eines Zustands, der durch einen Peroxisom-Proliferator-aktivierten Rezeptor (PPAR) moduliert wird.

13. Verwendung nach Anspruch 12, worin der Peroxisom-Proliferator-aktiverte Rezeptor für einen selektiv modulierten PPAR α steht.

14. Verwendung einer Verbindung nach einem der Ansprüche 1 bis 10 zur Herstellung eines Arzneimittels für die Behandlung oder Prävention von Diabetes mellitus bei einem Säuger.

15. Verwendung einer Verbindung nach einem der Ansprüche 1 bis 10 zur Herstellung eines Arzneimittels für die Behandlung von Syndrom X bei einem Säuger.

16. Verwendung einer Verbindung nach einem der Ansprüche 1 bis 10 zur Herstellung eines Arzneimittels für die Behandlung oder Prävention einer Kardiovaskularkrankheit bei einem Säuger.

17. Verwendung nach Anspruch 16, worin die Kardiovaskularkrankheit Atherosklerose ist.

## Revendications

1. Composé répondant à la formule I: 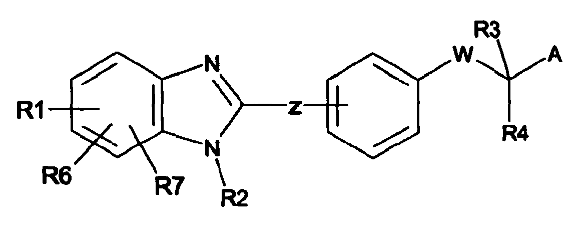 et les sels pharmaceutiquement acceptables de ce composé, dans lequel :
a) R1 est choisi parmi le groupe constitué d'un atome d'hydrogène, des groupes alkyle en C₁-C₈, aryl(alkyle en C₀-C₄), hétéroaryl(alkyle en C₀-C₄), et (cycloalkyle en C₃-C₆)aryle(alkyle en C₀-C₂), dans lequel lesdits groupes alkyle en C₁-C₈, aryl(alkyle en C₀-C₄), hétéroaryl(alkyle en C₀-C₄), et (cycloalkyle en C₃-C₆) aryle(alkyle en C₀-C₂) sont chacun éventuellement substitués avec un à trois substituants chacun indépendamment choisis parmi R1';
b) R1', R2', R4', R6', A', Z' et R19' représentent chacun le groupe constitué des groupes alkyle en C₁-C₅, alcoxy en C₁-C₅, halogénoalkyle en C₁-C₅, halogénoalcoxy en C₁-C₅, nitro, cyano, CHO, hydroxyle, (acide alcanoïque en C₁-C₄)phényle, aryloxy, SO₂R16, SR5, benzyloxy, alkylcarboxamido et COOH ;
c) R2 est choisi parmi le groupe constitué d'un atome d'hydrogène, (alkyle en C₂-C₄)-O-(alkyle en C₂-C₄)-O-(alkyle en C₁-C₄), alkylène en C₁-C₈, aryl(alkyle en C₀-C₄), hétéroaryl(alkyle en C₀-C₄), et (cycloalkyle en C₃-C₆)(alkyle en C₀-C₄), et dans lequel lesdits groupes (alkyle en C₂-C₄)-O-(alkyle en C₂-C₄)-O-(alkyle en C₁-C₄), alkylène en C₁-C₈, aryl(alkyle en C₀-C₄), hétéroaryl(alkyle en C₀-C₄, et (cycloalkyle en C₃-C₆)(alkyle en C₀-C₄) sont chacun éventuellement substitués avec un à trois substituants chacun indépendamment choisis parmi R2' ;
d) R3 est choisi parmi le groupe constitué d'un atome d'hydrogène, des groupes alkyle en C₁-C₅, et alcoxy en C₁-C₅;
e) R4 est choisi parmi le groupe constitué d'un atome d'hydrogène, des groupes alkyle en C₁-C₅, alcoxy en C₁-C₅, cycloalkyle en C₃-C₆, et aryl(alkyle en C₀-C₄), et dans lequel lesdits groupes alkyle en C₁-C₅, alcoxy en C₁-C₅, cycloalkyle en C₃-C₆, et aryl(alkyle en C₀-C₄) sont chacun éventuellement substitués avec un à trois substituants chacun indépendamment choisis parmi R4' ; et dans lequel R3 et R4 sont éventuellement combinés pour former un groupe cycloalkyle en C₃-C₄ ;
f) R5 et R16 sont chacun choisis parmi le groupe constitué d'un atome d'hydrogène, des groupes alkyle en C₁-C₆ et halogénoalkyle en C₁-C₆;
g) R6 et R7 sont chacun indépendamment choisis parmi le groupe constitué d'un atome d'hydrogène, des groupes alkyle en C₁-C₆, alcényle en C₁-C₆, halogénoalkyle en C₁-C₆, d'un atome d'halogène, des groupes oxy, alcoxy en C₁-C₆, et dans lequel lesdits groupes alkyle en C₁-C₆, halogénoalkyle en C₁-C₆, et alcoxy en C₁-C₆ sont chacun éventuellement substitués avec un à trois substituants chacun indépendamment choisis parmi R6' ; et dans lequel R6 et R7 se combinent éventuellement pour former un groupe aryle en C₃-C₆ qui est condensé avec le groupe duquel R6 et R7 sont chacun originaires ;
h) W est choisi parmi le groupe constitué de O, C, N et S ;
i) Z représente un groupe alkyle en C₃ éventuellement substitué avec Z' ;
j) A est choisi parmi le groupe constitué des groupes carboxyle, carboxamide, sulfonamide, acylsulfonamide, tétrazole, et (CH₂)ₙCOOR19, et dans lequel lesdits groupes sulfonamide, acylsulfonamide, et tétrazole sont chacun éventuellement substitués avec un à trois substituants chacun indépendamment choisis parmi A';
k) n vaut 0, 1, 2 ou 3; et
l) R19 est choisi parmi le groupe constitué d'un atome d'hydrogène, des groupes alkyle en C₁-C₄ et arylméthyle, dans lequel lesdits groupes alkyle et arylméthyle sont chacun éventuellement substitués avec un à trois substituants chacun indépendamment choisis parmi R19'.

2. Composé selon la revendication 1, dans lequel W représente O.

3. Composé selon l'une quelconque des revendications 1 ou 2, dans lequel A représente COOH.

4. Composé selon l'une quelconque des revendications 1, 2, ou 3, dans lequel Z est non substitué.

5. Composé selon l'une quelconque des revendications 1, 2, 3, ou 4, dans lequel R6 et R7 représentent chacun un groupe alkyle en C₁-C₂.

6. Composé selon l'une quelconque des revendications 1, 2, 3 ou 4, dans lequel R6 et R7 se combinent pour former un groupe aromatique cyclique condensé à 6 chaînons.

7. Composé selon l'une quelconque des revendications 1, 2, 3 ou 4, dans lequel R1 représente un groupe phényle.

8. Composé selon l'une quelconque des revendications 1, 2, 3, 4, 5, 6, ou 7, dans lequel R2 représente un groupe alkyle en C₁-C₆ linéaire ou ramifié.

9. Composé selon l'une quelconque des revendications 1, 2, 3, 4, 5, 6, ou 7, dans lequel R2 représente un groupe (alkyle en C₁-C₃)phényle ou (alkyle en C₁-C₃)naphtyle.

10. Composé selon la revendication 9, dans lequel le groupe phényle ou naphtyle est substitué avec un ou deux substituants indépendamment choisis parmi le groupe constitué d'un groupe alkyle en C₁-C₃, d'un atome d'halogène, et d'un groupe alcoxy en C₁-C₃.

11. Composition pharmaceutique, comprenant un support pharmaceutiquement acceptable et au moins un composé selon l'une quelconque des revendications 1 à 10.

12. Utilisation d'un composé selon l'une quelconque des revendications 1 à 10, pour la fabrication d'un médicament destiné au traitement d'une pathologie modulée par un récepteur activé par le proliférateur de peroxisome.

13. Utilisation selon la revendication 12, dans lequel le récepteur activé par le proliférateur de peroxisome est sélectivement modulé par le PPAR α.

14. Utilisation d'un composé selon l'une quelconque des revendications 1 à 10, pour la fabrication d'un médicament destiné au traitement ou à la prévention d'un diabète sucré chez un mammifère.

15. Utilisation d'un composé selon l'une quelconque des revendications 1 à 10, pour la fabrication d'un médicament destiné au traitement du syndrome X chez un mammifère.

16. Utilisation d'un composé selon l'une quelconque des revendications 1 à 10, pour la fabrication d'un médicament destiné au traitement ou à la prévention d'une maladie cardiovasculaire chez un mammifère.

17. Utilisation selon la revendication 16, dans lequel la maladie cardiovasculaire est l'athérosclérose.
